# EUROPEAN PATENT APPLICATION

(11) **EP 4 778 918 A1**
(43) Date of publication of application: **22.07.2026**
(21) Application number: 24865574.8
(22) Date of filing: 13.09.2024
(51) Int. Cl.: C07D 239/96, A61K 51/04

(54) **RADIOACTIVE POSITRON-EMITTING NUCLIDE CARBON-11 LABELED COMPOUND AND RADIOACTIVE COMPOSITION**

(30) Priority: 13.09.2023 JP 2023148793
(71) Applicant: The University of Osaka, Osaka 565-0871 (JP)
(72) Inventor: MATSUOKA, Ken, Suita-shi, Osaka 565-0871 (JP); TAKASHIMA, Seiji, Suita-shi, Osaka 565-0871 (JP); SEGAWA, Takatsugu, Suita-shi, Osaka 565-0871 (JP); WATABE, Tadashi, Suita-shi, Osaka 565-0871 (JP); NAKA, Sadahiro, Suita-shi, Osaka 565-0871 (JP)
(74) Representative: Marks & Clerk LLP
(86) International application number: PCT/JP2024/032966
(87) International publication number: WO 2025/058075

(57) **Abstract**

The present invention solves the problem of providing a compound that can act as a tracer to detect lymphocyte infiltration into myocardial tissue using positron emission tomography (PET) or the like.

The problem is solved by a radioactive positron-emitting nuclide carbon-11 labeled compound ([¹¹C] labeled compound) represented by the following general formula (I), or a pharmaceutically acceptable salt thereof: 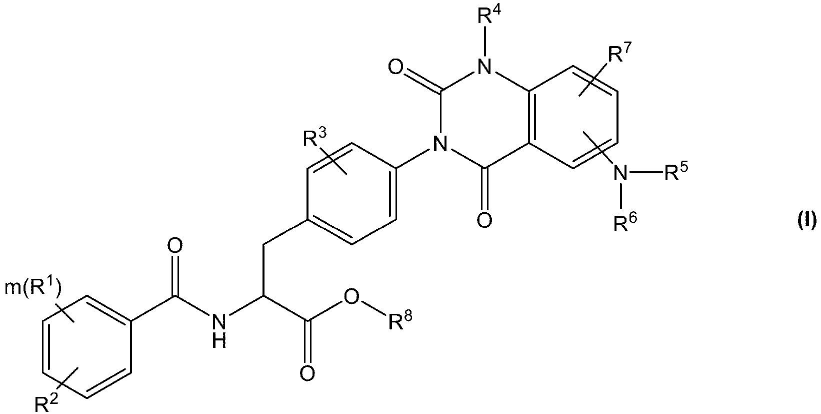 (wherein,
R¹ is a chlorine atom.
m is an integer from 1 to 3.
At least one of R², R³, R⁴, R⁵, R⁶, and R⁷ is a linear or branched alkyl group having 1 to 6 carbon atoms, the alkyl group has radioactive positron-emitting nuclide carbon-11 [¹¹C], and the rest are hydrogen atoms.
R⁸ is a hydrogen atom or a linear or branched alkyl group having 1 to 6 carbon atoms.)

## Description

### Technical Field

The present description discloses a radioactive positron-emitting nuclide carbon-11 labeled compound or a pharmaceutically acceptable salt thereof, and a radioactive composition.

### Background Art

Myocarditis is an inflammatory disease primarily affecting myocardium. Myocarditis accounts for approximately 10% of sudden death, and approximately 30% of cardiomyopathy cases progress to chronic myocarditis (dilated cardiomyopathy). Mortality due to myocarditis is 22%, and among myocarditis cases, the mortality due to fulminant myocarditis is as high as 43%. According to Non Patent Literature 1, myocarditis is classified into lymphocytic myocarditis, giant cell myocarditis, eosinophilic myocarditis, and granulomatous myocarditis based on histological characteristics. Lymphocytic myocarditis is often caused by viral infections such as coxsackievirus and adenovirus. It is believed that giant cell myocarditis, eosinophilic myocarditis and granulomatous myocarditis are often complications of cardiotoxic substances, drugs, autoimmunity, or systemic diseases. As for the drugs, it has been reported that anthracyclines, fluorouracil, immune checkpoint inhibitors, COVID-19 vaccines, and others cause myocarditis. Regarding immune checkpoint inhibitors, the incidence rate of myocarditis was 0.5% in subjects administered with anti-PD-1 antibody alone, 2.4% in subjects administered with anti-PD-L1 antibody alone, and 3.3% in subjects administered with anti-CTLA4 antibody alone. In foci of myocarditis thought to be caused by immune checkpoint inhibitors, tissue infiltration with a predominance of T lymphocytes has been identified. Furthermore, when myocarditis is caused by administration of immune checkpoint inhibitors, the mortality is 46% when the mortality due to heart failure and the mortality due to cardiogenic causes are combined (Non Patent Literature 2).

### Citation List

### Non Patent Literature

[Non Patent Literature 1] Kociol. R. D. et al: Circulation. 2020;141
[Non Patent Literature 2] Mahmood, SS: J Am Coll Cardiol 2018;71:1755-64

### Summary of Invention

### Technical Problem

Particularly, in fulminant myocarditis, disease state progresses rapidly, causing hemodynamic collapse, and if myocardial damage in acute phase is severe, cardiac function does not recover even after inflammation is ameliorated, and it shifts to dilated cardiomyopathy, resulting in worse prognosis. Therefore, prompt diagnosis of myocarditis and initiation of treatment with this drug as soon as possible is desirable.

To diagnose myocarditis and select treatment, it is necessary to observe the myocardial tissue by pathological examination with myocardial biopsy. However, myocardial biopsy is highly invasive and may cause complications such as cardiac tamponade. In addition, since the biopsy site is confined to the right ventricular septal wall, the risk of false negatives is also high.

An object of the present invention is to provide a compound that can act as a tracer to detect lymphocyte infiltration into myocardial tissue using positron emission tomography (PET) or the like.

### Solution to Problem

The present invention may include the following embodiments.
Item 1. A radioactive positron-emitting nuclide carbon-11 labeled compound ([¹¹C] labeled compound) represented by the following general formula (I), or a pharmaceutically acceptable salt thereof: 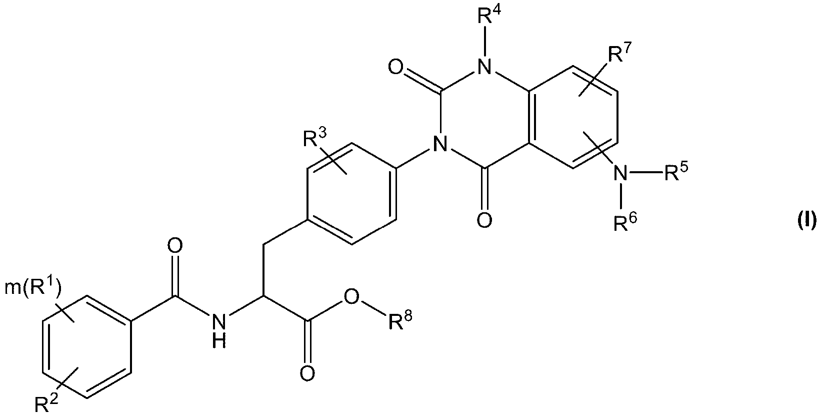 (wherein,
   R¹ is a chlorine atom.
   m is an integer from 1 to 3.
   At least one of R², R³, R⁴, R⁵, R⁶, and R⁷ is a linear or branched alkyl group having 1 to 6 carbon atoms, wherein the alkyl group has a radioactive positron-emitting nuclide carbon-11 [¹¹C], and the rest are hydrogen atoms.
   R⁸ is a hydrogen atom or a linear or branched alkyl group having 1 to 6 carbon atoms.)
Item 2. The [¹¹C] labeled compound or the pharmaceutically acceptable salt thereof according to item 1, wherein the [¹¹C] labeled compound is represented by the following general formula (II): 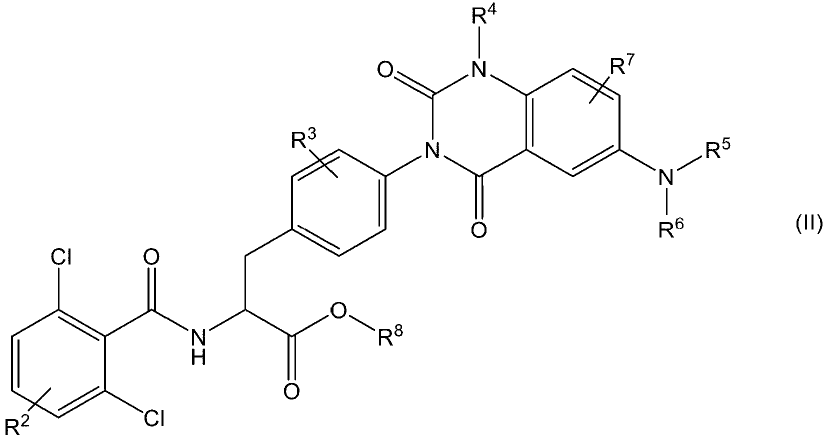 (wherein,
   R², R³, R⁴, R⁵, R⁶, R⁷ and R⁸ are the same as described above.)
Item 3. The [¹¹C] labeled compound or the pharmaceutically acceptable salt thereof according to item 1, wherein the [¹¹C] labeled compound is represented by the following general formula (1) or (2): 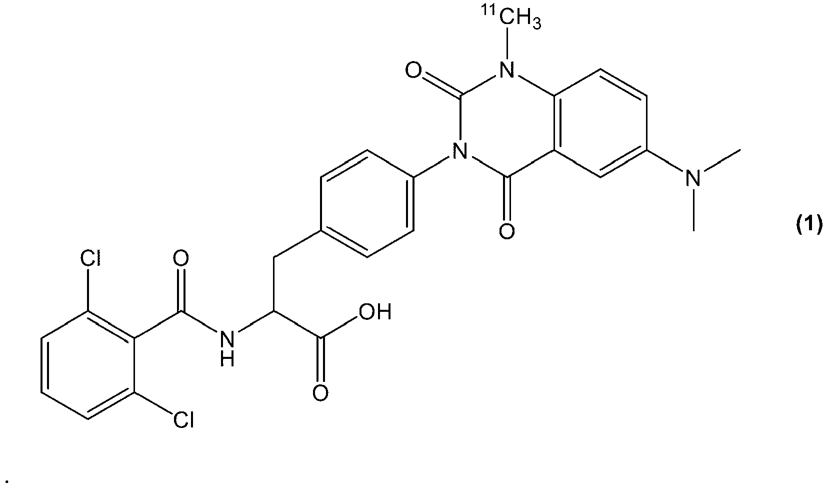 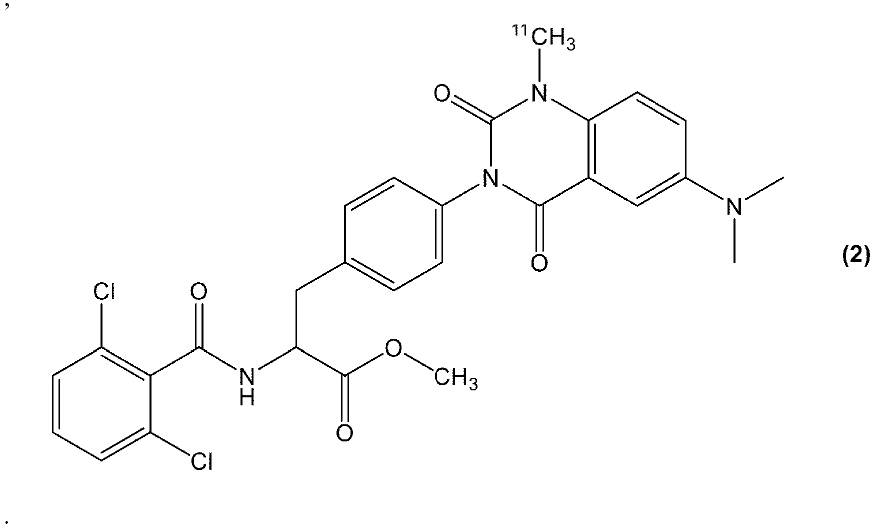
Item 4. A radioactive composition comprising the [¹¹C] labeled compound or the pharmaceutically acceptable salt thereof according to item 1.
Item 5. The radioactive composition according to item 4, used for detecting a lymphocytic inflammatory disease.
Item 6. The radioactive composition according to item 5, wherein the lymphocytic inflammatory disease is myocarditis or myelitis (provided that autoimmune myocarditis with myosin as an antigen is excluded from the myocarditis).
Item 7. The radioactive composition according to item 5, wherein the myocarditis is myocarditis that develops in a manner dependent on binding of Vcam-1 and VLA-4.
Item 8. The radioactive composition according to item 5, wherein the myocarditis is myocarditis caused by an immune checkpoint inhibitor.
Item 9. The radioactive composition according to item 4, used for determining a therapeutic effect against myocarditis.
Item 10. The radioactive composition according to item 4, used for predicting a therapeutic effect against myocarditis.
Item 11. The radioactive composition according to item 4, used in patients with myocarditis or patients suspected of having myocarditis to predict a therapeutic effect of treatment with a VLA-4 inhibitor and to determine whether to administer said treatment to patients in whom said treatment is determined to be effective.

### Advantageous Effects of Invention

A compound that can act as a tracer to detect lymphocyte infiltration into myocardial tissue using positron emission tomography (PET) or the like, can be provided.

### Brief Description of Drawings

FIG. 1 shows a production scheme of [¹¹C]HCA2969.
FIG. 2 shows a chromatogram of a produced [¹¹C]HCA2969.
FIG. 3 shows the stability of the produced [¹¹C]HCA2969 over time.
FIG. 4 shows macro images and low-power field and high-power field images of hematopoietic-stained tissue of a heart on Day 12, Day 19, and Day 25.
FIG. 5 shows results of population analysis of cells in cardiac muscle tissue by single-cell analysis.
FIG. 6 shows expressions of Vcam-1 in a CTL and on day 8, day 14, day 21, and day 25.
FIG. 7A shows a schedule of an adhesion experiment. FIG. 7B shows a result of Western blotting for expression of Vcam-1. FIG. 7C shows TNFα-dependent adhesiveness between cardiac fibroblasts and monocytes.
FIG. 8A shows a schedule of an experiment. FIG. 8B shows an effect of siVcam-1 on suppressing Vcam-1 gene expression. FIG. 8C shows adhesiveness between cardiac fibroblasts and monocytes.
FIG. 9A shows an administration schedule of a Vcam-1-neutralizing antibody and a VLA-4-neutralizing antibody. FIG. 9B shows left ventricular fractional shortening (% fractional shortening; FS) measured by echocardiography in all mice in a CTL group and a neutralizing antibody-administered group. FIG. 9C shows boxplots of left ventricular end-diastolic diameter and left ventricular fractional shortening on day 28 in the same mice as in FIG. 9B. FIG. 9D shows Kaplan-Meier curves for the CTL group and the neutralizing antibody-administered group.
FIG. 10A shows an administration schedule of the neutralizing antibody. FIG. 10B shows Kaplan-Meier curves for each group.
FIG. 11A shows %FS values of wild-type mice (WT), F1 obtained by crossing wild-type mice with MRL-Pdcd1⁻/⁻ mice (hetero: MRL-Pdcd1⁻), and MRL-Pdcd1⁻/⁻ mice (homo) at 3 weeks after birth. FIG. 11B shows results of dividing the homo group into a high %FS group (good cardiac function group, n=11) and a low %FS group (reduced cardiac function group, n=20). FIG. 11C shows an administration schedule of the VLA-4-neutralizing antibody.
FIG. 12 shows clinical data for a CTL group, a VLA-4 group, and a normal EF group.
FIG. 13 shows Kaplan-Meier curves for the CTL group (sign "a") and the VLA-4 group (sign "b").
FIG. 14 shows Kaplan-Meier curves for the CTL group (sign "a"), the normal EF group (sign "b") and the VLA-4 group (sign "c").
FIG. 15 shows Kaplan-Meier curves for each group when a VLA4 inhibitor was administered to the MRL-Pdcd1⁻/⁻ mice. In the figure, the sign "a" denotes an AJM300(+) group, and the sign "b" denotes an AJM300(-) group.
FIG. 16 shows results of heart rate and left ventricular contractile performance: % Fractional Shortening (%) for each group when the VLA4 inhibitor was administered to the MRL-Pdcd1-/- mice.
FIG. 17 shows levels of soluble Vcam-1 in sera of three healthy subjects (CTL), four patients with Covid-related myocarditis (Covid related), eight patients with lymphocytic myocarditis (Lym), six patients with eosinophilic myocarditis (Eosi), and three patients with giant cell myocarditis (Giant).
FIG. 18 shows levels of sVcam-1 in sera of the patients with lymphocytic myocarditis in acute and convalescent phases.
FIG. 19A shows the radiation dose at each addition amount of [¹¹C]HCA2969. FIG. 19B shows the radiation dose per 1 nM of [¹¹C]HCA2969.
FIG. 20 shows PET/CT images obtained using [¹¹C]HCA2969 in two MRL-Pdcd1-/- mice.
FIG. 21 shows PET/CT images obtained using [¹¹C]HCA2969 in two wild-type mice.
FIG. 22 shows PET/CT images obtained using [¹¹C]HCA2969 in an autoimmune myocarditis model rat.
FIG. 23 shows a schedule of an anti-VLA-4-neutralizing antibody administration experiment in a rat autoimmune myocarditis model.
FIG. 24 shows a schedule of an AJM300 administration experiment in a rat autoimmune myocarditis model.
FIG. 25A shows left ventricular contractile performance for a placebo group, an AJM300-administered group, and a neutralizing antibody-administered group. FIG. 25B shows the distribution of heart rate in the placebo group, the AJM300-administered group, and the neutralizing antibody-administered group.
FIG. 26A shows an example of PET/CT imaging for a wild-type mouse (WT) and a PD1-/- mouse (PD1-/-). FIG. 26 B is a boxplot quantifying the accumulation in a cardiac region for each group.
FIG. 27A shows an image of myocardial tissue from an individual marked with a solid circle in the boxplot of the placebo group in FIG. 26B. FIG. 27B shows an image of myocardial tissue from the individual marked with a dotted circle in the boxplot of the placebo group in FIG. 26B.
FIG. 28 shows SUV median (Bq/ml) and SUV mean (Bq/ml) for a negative control (Control) group and an EAE model (EAE) group. FIG. 28A shows the SUV median (Bq/ml), and FIG. 28B shows the SUV mean (Bq/ml).
FIG. 29 shows measurement results from a well counter.
FIG. 30 shows %ID/g of individuals in an EAE model group and a negative control group (CTL group).
FIG. 31 shows images of HE-stained spinal cords of individuals of the EAE model group.

### Description of Embodiments

### 1. Radioactive positron-emitting nuclide carbon-11 labeled compound, or a pharmaceutically acceptable salt thereof

One embodiment relates to a radioactive positron-emitting nuclide carbon-11 labeled compound (hereinafter also referred to as "[¹¹C] labeled compound"), or a pharmaceutically acceptable salt thereof.

The [¹¹C] labeled compound is a compound represented by the following general formula (I), or a pharmaceutically acceptable salt thereof: 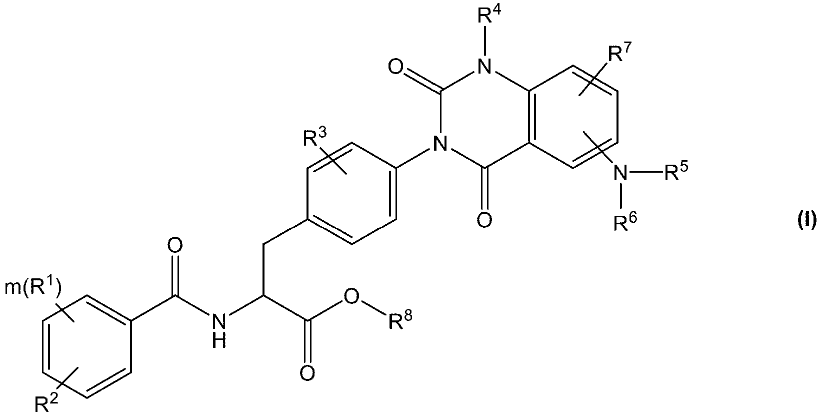 (wherein,
R¹ is a chlorine atom.
m is an integer from 1 to 3.
At least one of R², R³, R⁴, R⁵, R⁶, and R⁷is a linear or branched alkyl group having 1 to 6 carbon atoms, wherein the alkyl group has a radioactive positron-emitting nuclide carbon-11 [¹¹C], and the rest are hydrogen atoms.
R⁸ is a hydrogen atom or a linear or branched alkyl group having 1 to 6 carbon atoms.)

The m is preferably 2.

The linear or branched alkyl group having 1 to 6 carbon atoms in the definitions of R², R³, R⁴, R⁵, R⁶, R⁷, and R⁸ may include, for example, methyl group, ethyl group, n-propyl group, isopropyl group, n-butyl group, sec-butyl group, isobutyl group, tert-butyl group, n-pentyl group, neopentyl group, isopentyl group, sec-pentyl group, tert-pentyl group, n-hexyl group, 2-methylpentyl group, 3-methylpentyl group, 2, 3-dimethylbutyl group, and 2, 2-dimethylbutyl group.

The linear or branched alkyl group having 1 to 6 carbon atoms is preferably a methyl group, an ethyl group, an n-propyl group or an isopropyl group, and more preferably a methyl group.

The [¹¹C] is preferably a carbon of a methyl group.

It is preferable that any one, any two, or all of the R², R³, and R⁷ are hydrogen atoms.

The R⁸ is preferably a hydrogen atom or a methyl group, and more preferably a hydrogen atom.

The [¹¹C] labeled compound is preferably a compound represented by the following general formula (II): 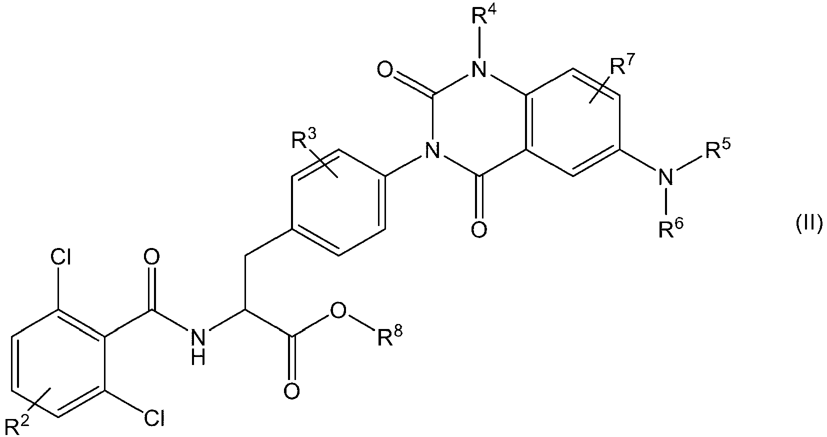 (wherein,
R², R³, R⁴, R⁵, R⁶, R⁷ and R⁸ are the same as described above.)

The [¹¹C] labeled compound is more preferably a compound represented by the following formula (1) or (2):

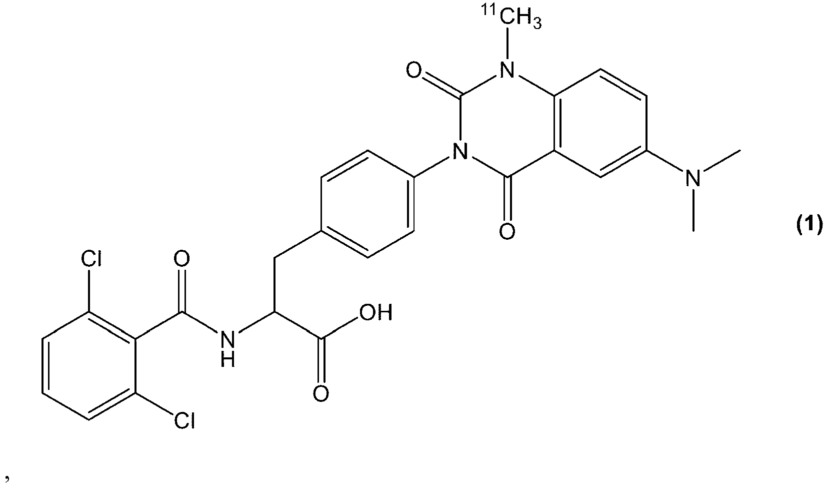

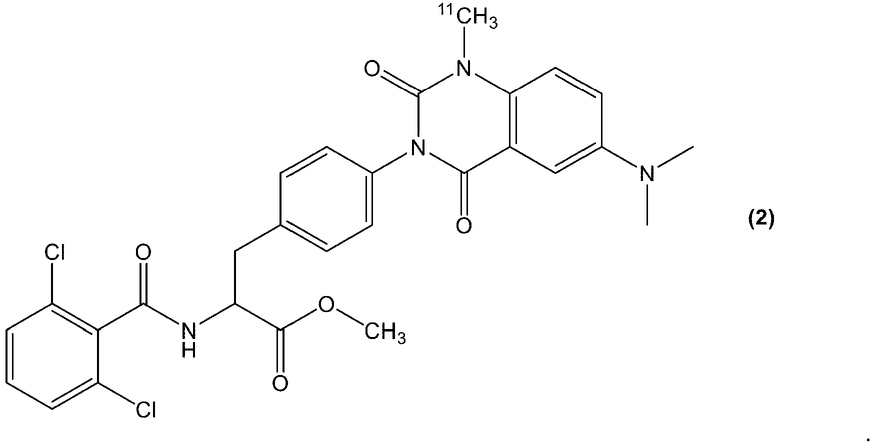

Salts of the [¹¹C] labeled compound may include, for example, inorganic acid salts such as hydrochloride, nitrate, hydrobromide and sulfate; organic carboxylate salts such as acetate and trifluoroacetate; sulfonate salts such as methanesulfonate; or metal salts such as sodium salt, potassium salt, calcium salt, and magnesium salt; salts of basic substance such as ammonium salt.

### 2. Method for Producing [¹¹C] labeled compound

The [¹¹C] labeled compound can be produced according to the following production scheme.

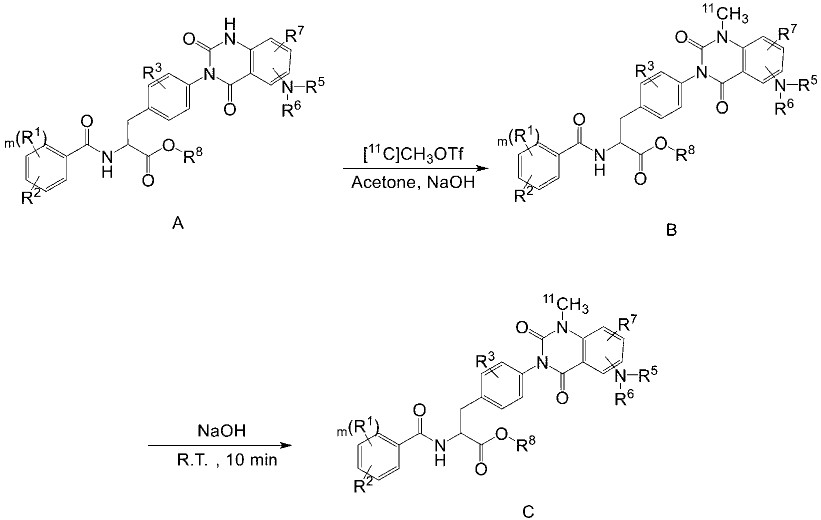

In the above scheme, R¹, m, R², R³, R⁴, R⁵, R⁶, R⁷, and R⁸ are the same as in the general formula (I).
(1) For example, using a medical cyclotron (CYPRIS HM-18, Sumitomo Heavy Industries, Ltd.), [¹¹C]CO₂ is produced via nuclear reaction of ¹⁴N(p,α)¹¹C using N₂ gas as a target. The obtained [¹¹C]CO₂ is introduced into a vapor phase method methyl iodide synthesizer (C-GPC-100, Sumitomo Heavy Industries, Ltd.) to synthesize [¹¹C] trifluoromethanesulfonate ([¹¹C]CH₃OTf) having high reaction activity.
(2) The [¹¹C]CH₃OTf is introduced into a reaction vessel containing an acetone solution in which Compound A has been dissolved to a concentration of, for example, about 0.2 mg/mL to 1.5 mg/mL and a base such as sodium hydroxide at a final concentration of about 0.003 mol/L to 0.008 mol/L have been added and subjected to bubbling. This reaction yields Compound B.
(3) A base such as sodium hydroxide is added to the solution obtained after the bubbling in the above step (2) so that the final concentration is about 0.01 mol/L to 0.03 mol/L, and a deprotection reaction is performed at room temperature (about 20°C to 27°C) for about 10 minutes. This reaction yields Compound C.
(4) [¹¹C]HCA2969 is fractionated and collected by HPLC from the solution obtained after the deprotection in the above step (3).

Preferably, the [¹¹C] labeled compound can be produced according to the following production scheme.

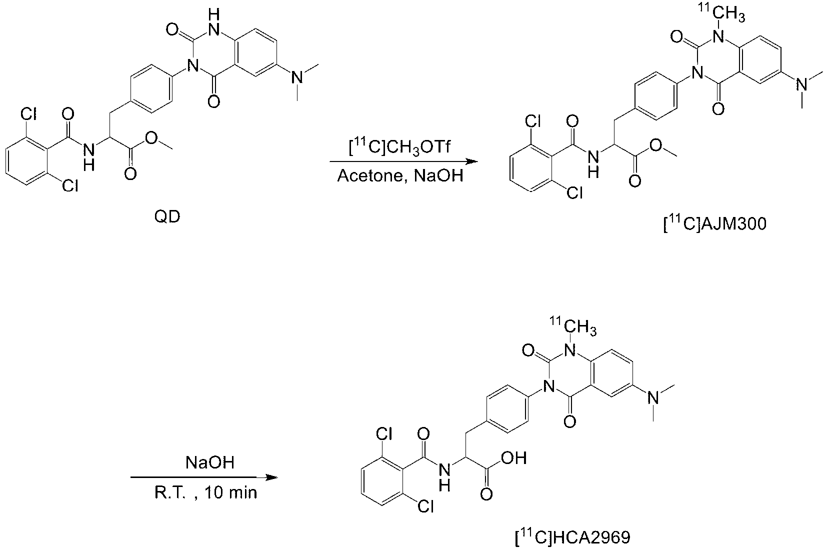

In this scheme, by using compound QD as a starting material as a preferred embodiment of the compound A in step (2) of the above production method, [¹¹C]AJM300, which is a preferred embodiment of the compound B is obtained in step (2). Subsequently, in step (3), [¹¹C]HCA2969, which is a preferred embodiment of the compound C, is obtained.

### 3. Radioactive composition containing [¹¹C] labeled compound or containing pharmaceutically acceptable salt thereof

One embodiment relates to a radioactive composition containing a [¹¹C] labeled compound or containing a pharmaceutically acceptable salt thereof (hereinafter also referred to simply as a "radioactive composition").

The radioactive composition may be in a dry state or in a liquid state dissolved in a solvent. The solvent may include ethanol, a mixture of ethanol and water (where the ethanol content is greater than 0% but less than 99.5%), N,N-dimethylformamide, and the like.

The radioactive composition may be administered at a dose of about 1 MBq/kg to 500 MBq/kg, preferably about 3 MBq/kg to 100 MBq/kg, in terms of radiation dose.

The radioactive composition may be used to detect a lymphocytic inflammatory disease. The lymphocytic inflammatory disease is preferably a lymphocytic inflammatory disease for which a VLA4 inhibitor is effective. More preferably, the lymphocytic inflammatory disease may include myocarditis, encephalomyelitis, ulcerative colitis, Crohn's disease, and heart transplant rejection. Even more preferably, the lymphocytic inflammatory disease may include myocarditis and encephalomyelitis.

In the present description, "myocarditis" is an inflammatory disease primarily affecting myocardium. Myocarditis includes acute myocarditis and chronic myocarditis according to a classification based on clinical course. In addition, acute myocarditis includes fulminant myocarditis and myocarditis other than fulminant myocarditis. Chronic myocarditis includes dilated cardiomyopathy with ventricular hypertrophy and chronic myocarditis other than dilated cardiomyopathy.

In addition, myocarditis includes lymphocytic myocarditis, giant cell myocarditis, eosinophilic myocarditis, granulomatous myocarditis, and the like according to a histological classification. It has been reported that lymphocytic myocarditis is caused by viral infection. It is believed that most cases are caused by viral infection due to pathogenic virus such as enteroviruses including coxsackie virus (group A or group B), echovirus, poliovirus, and the like; hepatitis virus (type A or type C); influenza virus (type A or type B); respiratory syncytial virus; mumps virus; measles virus; dengue virus; yellow fever virus; chikungunya virus; rabies virus; HIV virus; vaccinia virus; herpes zoster virus; cytomegalovirus; herpes simplex virus; EB virus; measles virus; adenovirus; parvovirus, and the like. It has been reported that giant cell myocarditis, eosinophilic myocarditis, and giant cell myocarditis are caused by bacterial infection. It has been reported that eosinophilic myocarditis is caused by fungal infection. It is believed that granulomatous myocarditis is often associated with infectious diseases such as rickettsial infection, spirochete infection, protozoan infection, and parasitic infection; ingestion of drugs and chemicals; allergies and autoimmune diseases; Kawasaki disease; sarcoidosis; radiation exposure; heatstroke, and others. The drugs that may cause myocarditis may include anthracyclines, fluorouracil, immune checkpoint inhibitors, coronavirus vaccine, and the like. Immune checkpoint inhibitors may include anti-PD-1 antibody, anti-PD-L1 antibody, and anti-CTLA4 antibody.

The myocarditis preferably does not include autoimmune myocarditis with myosin as an antigen. In addition, the myocarditis preferably does not include eosinophilic myocarditis.

The myocarditis is preferably lymphocytic myocarditis. In addition, the myocarditis is preferably myocarditis which develops in a manner dependent on the binding between Vcam-1 and VLA-4. More preferably, the myocarditis is caused by an immune checkpoint inhibitor.

As used herein, VLA-4 is a protein also referred to as integrin subunit alpha 4, or CD49D, which in the case of humans is expressed from a gene registered in the National Center for Biotechnology Information as Gene ID: 3676. VLA-4 is a ligand for vascular cell adhesion molecule 1 (VCAM1; CD106; INCAM-100).

Detection of myocarditis using the radioactive composition employs [¹¹C] in the radioactive composition as a tracer in a body. [¹¹C] can be detected using positron emission tomography (hereinafter also referred to as "PET scan"). The PET scans can be performed according to known methods. The PET scans may include scans using only positron emission tomography, PET-CT scans combining PET with CT scans, PET-MRI scans combining PET with MRI, and the like.

The radioactive composition can be used to determine the therapeutic effect against myocarditis. The therapeutic effect can be determined by: (1) acquiring, at the first time point, a first PET scan image using the radioactive composition for a patient suspected of having myocarditis or a patient with myocarditis; (2) treating the patient for myocarditis; (3) acquiring, at a second time point after the first time point, a second PET scan image using the radioactive composition for the patient during or after treatment for myocarditis; (4) comparing accumulation of [¹¹C] in a cardiac region in the first PET scan image and the second PET scan image; (5) determining that the therapeutic effect is present when the accumulation of [¹¹C] in the second PET scan image is less than that in the first PET scan image; and/or determining that the therapeutic effect is not present when the accumulation of [¹¹C] in the second PET scan image is equal to or greater than that in the first PET scan image.

Additionally, in one embodiment, the radioactive composition can be used to predict a therapeutic effect with a VLA-4 inhibitor.

For example, when a PET scan image using the radioactive composition is acquired for a patient suspected of having myocarditis or for a patient with myocarditis, and if accumulation of [¹¹C] is observed in a cardiac region, it can be determined that treatment with a VLA-4 inhibitor will be effective for the patient. Furthermore, when a PET scan image using the radioactive composition is acquired for a patient suspected of having myocarditis or for a patient with myocarditis, and if no accumulation of [¹¹C] is observed in a cardiac region, it can be determined that treatment with a VLA-4 inhibitor is not effective for the patient.

In general, a definitive diagnosis of myocarditis is made by obtaining myocardial tissue by endomyocardial biopsy and examining an image of the myocardial tissue. Thus, a patient with myocarditis refers to a patient with a definitive diagnosis of myocarditis. In addition, a patient suspected of having myocarditis refers to a patient in whom, although a definitive diagnosis has not been made, the possibility of myocarditis is suggested by electrocardiography; pulse rate; echocardiography (tomographic imaging; evaluation of cardiac function such as left ventricular fractional shortening, left ventricular ejection fraction, etc.); measurement of blood levels of CRP, AST, LDH, CK-MB, cardiac troponin (T or I), sVcam-1; chest X-ray; cardiac MRI, etc.

Treatment of myocarditis may include, for example, administration of a VLA-4 inhibitor, discontinuation of a drug causing myocarditis, and the like.

The VLA-4 inhibitor may include, for example, an anti-VLA-4 antibody or a fragment containing an antigen-binding domain thereof.

In the present description, the "anti-VLA-4 antibody" is not limited as long as it can specifically bind to a VLA-4 protein and inhibit functional expression thereof. Said antibody may be either a polyclonal antibody or a monoclonal antibody. Both the polyclonal antibody and the monoclonal antibody can be appropriately prepared by a method known to a person skilled in the art. When said antibody is a monoclonal antibody, the antibody may be a chimeric antibody, a humanized antibody or a human antibody prepared by a known method. The antibody may also be an antibody fragment such as Fab, F(ab)₂, diabody, scFv, minibody, peptibody, mimetibody, and the like. Preferably, the anti-VLA-4 antibody is a neutralizing antibody. For example, an anti-VLA-4 antibody may include InVivoMAb anti-mouse/human VLA-4 (CD49d) (Clone No. PS/2, Cat No. BE0071, Bio X Cell) and Vedolizumab described in literature: Ferreira, E.F.B. et al. (Current Medicinal Chemistry, 2021, 28, 5884-5895), and the like.

In the present description, the VLA-4 antagonist may be a type that competitively inhibits the VLA-4 (competitive inhibition type) or a type that noncompetitively inhibits the VLA-4 (noncompetitive inhibition type). The VLA-4 antagonist is preferably a noncompetitive inhibition type. The VLA-4 antagonist may be a compound or peptide.

The VLA-4 antagonist may include, for example, carotegrast methyl (literature: WO2016/051828A1); SB683699/Firategrast, R411/valategrast, CT7758/CDP323, DS-70, and TBC3486 described in literature: Ferreira, E.F.B. et al (Current Medicinal Chemistry, 2021, 28, 5884-5895); PN-943 and MORF-057 described in literature: Slack, RJ (Nature Reviews Drug Discovery volume 21, pages 60-78 (2022)); BIO1211 and BIO5192 described in literature: Baiula, M et al (Front Chem. 2019 Jul 9;7:489); ER464195-01 described in literature: Ohkuro, M et al (Nat Commun. 2018 May 17;9(1): 1982ER464195-01); E6007 which is an analog of the ER464195-01. The PN-943 is a peptide.

The VLA-4 inhibitor may include an RNA molecule targeting VLA-4 mRNA. The RNA molecule is not limited as long as it can target the VLA-4 mRNA and suppress an expression of the VLA-4 mRNA. The RNA molecule may include an RNA molecule that has an action to degrade a target mRNA, such as at least one selected from the group consisting of RNA molecules such as siRNA, shRNA, miRNA, and antisense RNA, and/or an RNA molecule that suppresses translation of the target mRNA. Sequences of these RNA molecules may be designed appropriately by a person skilled in the art using a known method based on information on a nucleotide sequence of above-mentioned gene that is the target. In addition, the RNA molecule may be prepared based on a known method, or commercially available RNA molecule may be used. For example, an antisense RNA for VLA-4 may include ATL1102 described in literature: Limmroth, V. et al (Neurology 83 November 11, 2014). The RNA molecule or a vector capable of expressing said RNA molecule may be included.

The vector capable of expressing the RNA molecule targeting the VLA-4 mRNA is not particularly limited as long as it can express the RNA molecule that suppresses the expression of said VLA-4 protein in the body of an individual or a cell. The vector may include, for example, a hairpin RNA expression vector and the like. The hairpin RNA expression vector contains at least a sense DNA nucleotide sequence that has the same sequence as the sense strand of the target mRNA (except that an uracil in the mRNA is replaced by a thymine) downstream of a promoter nucleotide sequence suitable for expression of small RNA such as, for example, U6 promoter; a loop nucleotide sequence that forms a loop structure after transcription; an antisense DNA nucleotide sequence that can bind complementarily in whole or in part to the sense DNA nucleotide sequence; and a terminator sequence. Examples of the vector may include a plasmid vectors, adeno-associated virus (AAV) vector, adenovirus vector, retroviral vector, lentiviral vector, and the like.

The VLA-4 inhibitor may include a genome editing system targeting VLA-4 gene. The genome editing system targeting VLA-4 gene is not limited as long as it is capable of causing recombination within VLA-4 gene in the body of an individual. The system may include, for example, CompoZr Zinc Finger Nuclease (ZFN) system, TAL effector nuclease (TALEN) system, Clustered regularly interspaced short palindromic repeats/CRISPR associated protein 9 (CRISPR/Cas9) system, and the like. The CRISPR/Cas9 system is preferred. A vector-based CRISPR/Cas9 system is preferred. In the vector-based CRISPR/Cas9 system, nucleic acids encoding CRISPR and nucleic acids encoding Cas9 may be on different vectors or on one vector. A promoter for allowing CRISPR to function is not particularly limited, and U6 promoter is preferred. A promoter for allowing Cas9 to function is not particularly limited, and a promoter expressed in mammalian cells, such as a cytomegalovirus promoter and the like, is preferred. Preferably as the CRISPR/Cas9 system, a commercially available vector, such as pX330-U6-Chimeric_BB-CBh-hSpCas9 vector and the like, may be used.

A sequence targeting VLA-4 gene that is to be incorporated into a CRISPR sequence (hereinafter also referred to as "VLA-4 gene-targeting sequence") is not limited as long as the sequence can be incorporated into a guide RNA (gRNA, also called crRNA) and transcribed by the CRISPR/Cas9 system to recombine VLA-4 gene. In general, it is believed that a sequence of around 20 bases in the 5' side upstream region of the nucleotide sequence "NGG" in VLA-4 gene can be selected as the VLA-4 gene-targeting sequence. The VLA-4 gene-targeting sequence may be designed using a known design tool published in Optimized CRISPR design tool (web page of Massachusetts Institute of Technology, ZhangLab (http://crispr.mit.edu/)), E-CRISP (http://www.e-crisp.org/E-CRISP/ (German Cancer Research Center)), ZiFiT Targeter(http://zifit.partners.org/ZiFit/ (Zing Finger consortium)), Cas9 design (http://cas9.cbi.pku.edu.cn (Peking University)), CRISPRdirect (http://crispr.dbcls.jp (University of Tokyo)), CRISPR-P (http://cbi.hzau.edu.cn/crispr/ (Huazhong Agricultural University)), and the like.

### Examples

Hereinafter, the present invention will be described in more detail with reference to examples. However, the present invention shall not be construed as limited to the examples.

In addition, all animal experiments in the examples were conducted according to Guide for the Care and Use of Laboratory Animals and were approved by the Animal Experiments Committee of The University of Osaka.

### 1. Production of [¹¹C]HCA2969

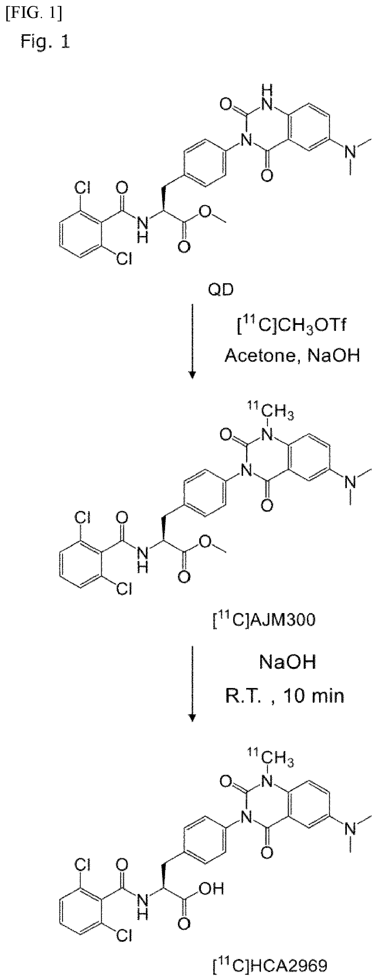

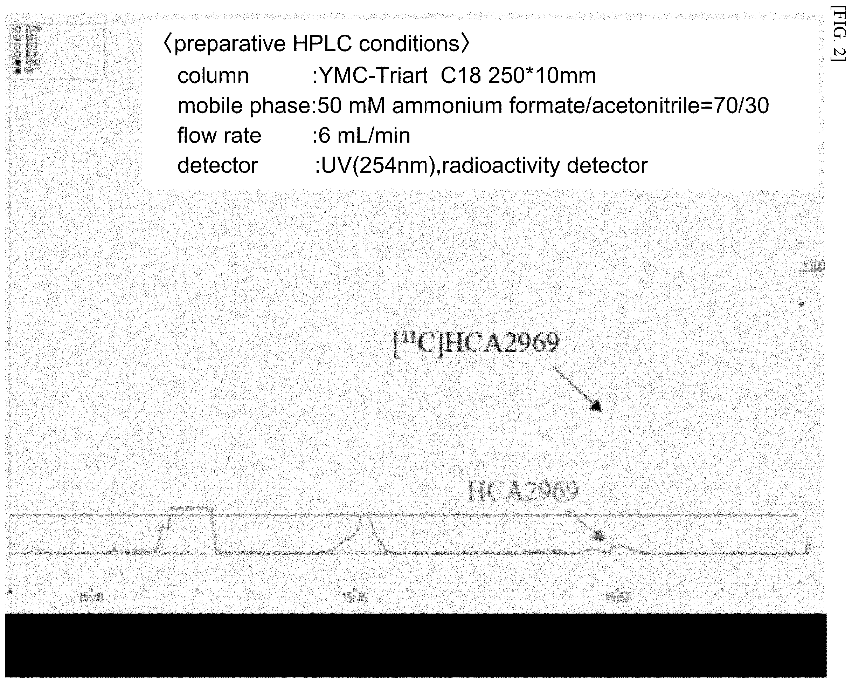

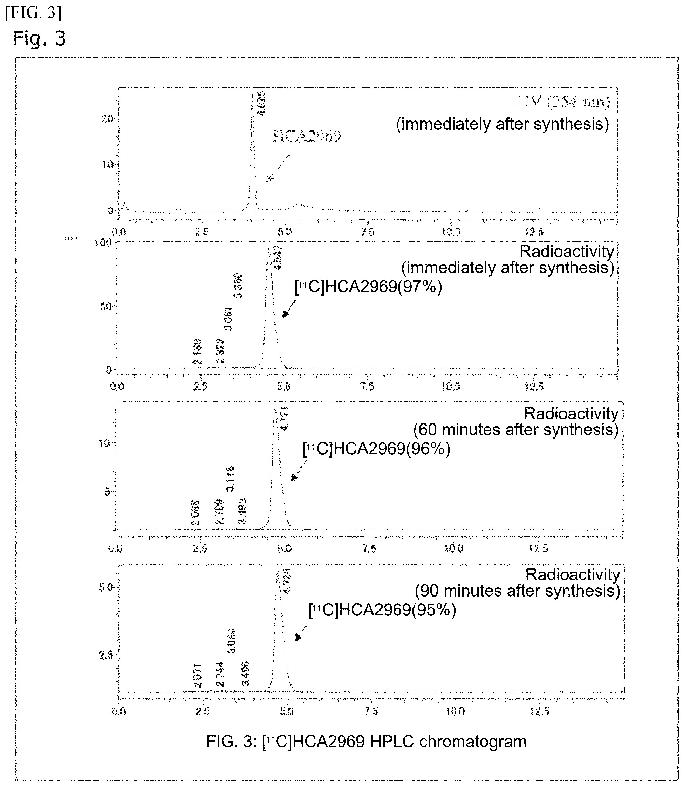

[¹¹C]HCA2969 was produced according to the following procedure. A synthesis scheme is shown in FIG. 1.
(1) [¹¹C]CO₂ was produced via nuclear reaction of ¹⁴N(p,α)¹¹C using N₂ gas as a target using a medical cyclotron (CYPRIS HM-18, Sumitomo Heavy Industries, Ltd.). The obtained [¹¹C]CO₂ was introduced into a vapor phase method methyl iodide synthesizer (C-GPC-100, Sumitomo Heavy Industries, Ltd.) to synthesize [¹¹C] trifluoromethanesulfonate ([¹¹C]CH₃OTf) having high reaction activity.
(2) [¹¹C]CH₃OTf was introduced into a reaction vessel containing 500 µL of a solution of 1 mg/mL HCA2969 precursor (QD) in acetone and 3 µL of 1 mol/L NaOH and subjected to bubbling.
(3) 100 µL of 0.1 mol/L NaOH was added to the solution obtained after the bubbling in the above step (2) and a deprotection reaction was performed at room temperature for 10 minutes.
(4) 1 mL of water for injection was added to the solution obtained after the deprotection in the above step (3), the mixture was mixed thoroughly, and the whole volume was injected into HPLC to fractionate and collect [¹¹C]HCA2969. A representative HPLC chromatogram is shown in FIG. 2.
(5) The fractionated and collected solution was diluted with water for injection, and the whole volume was introduced into a solid-phase extraction column (HLB column) to retain [¹¹C]HCA2969. The HLB column had been pre-conditioned with 10 mL of ethanol and 20 mL of water for injection.
(6) The HLB column retaining [¹¹C]HCA2969 in step (5) was washed with water for injection to remove impurities, and then [¹¹C]HCA2969 was recovered in a product vial with ethanol. The product vial previously contained physiological saline to achieve a final ethanol concentration of approximately 10%. [¹¹C]HCA2969 solution recovered in the product vial was used as a final formulation.
(7) A portion of the final formulation was extracted and subjected to a quality test. As a result of the quality test, it was confirmed that about 2 GBq of [¹¹C]HCA2969 was obtained using [¹¹C]CO₂, which was obtained under an irradiation condition of 25 µA for 30 minutes, as a starting material. In addition, it was confirmed that radiochemical purity of the [¹¹C]HCA2969 was stably high at 90% or more immediately after synthesis and even 90 minutes after synthesis. Results are shown in FIG. 3.

### 2. Single-cell analysis using autoimmune myocarditis model rat

### (i) Autoimmune myocarditis model rat

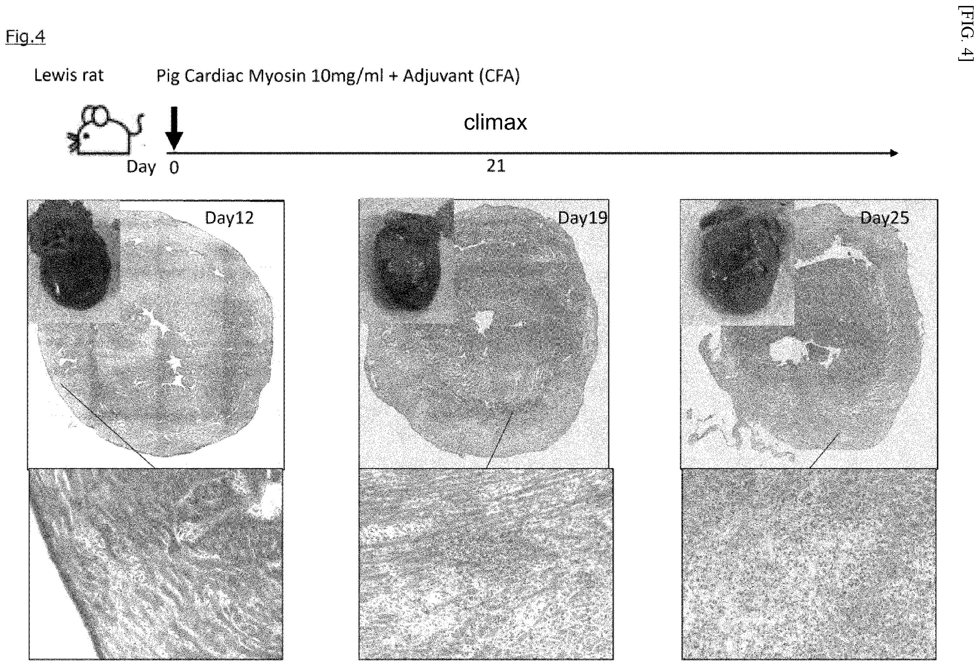

An autoimmune myocarditis model rat was prepared by administering 10 mg/ml of Pig Cardiac Myosin (purified in-house from pig heart) and Adjuvant (CFA 10 mg/ml; Chondrex, Inc., 7002) to Lewis rats (male, 8 weeks old). A day of administration of the Pig Cardiac Myosin was set as Day 0. In this model, myocarditis reached its climax on Day 21. FIG. 4 shows macro images and low-power field and high-power field images of hematopoietic-stained tissue of a heart on Day 12, Day 19, and Day 25. On Day 12, infiltration of inflammatory cells into cardiac muscle tissue began, and, on Day 19, more intense infiltration of the inflammatory cells was observed. On Day 25, destruction of the cardiac muscle tissue due to inflammation was observed. No infiltration of eosinophils was observed in any disease stage.

### (ii) Population analysis of cells in cardiac muscle tissue by single-cell analysis

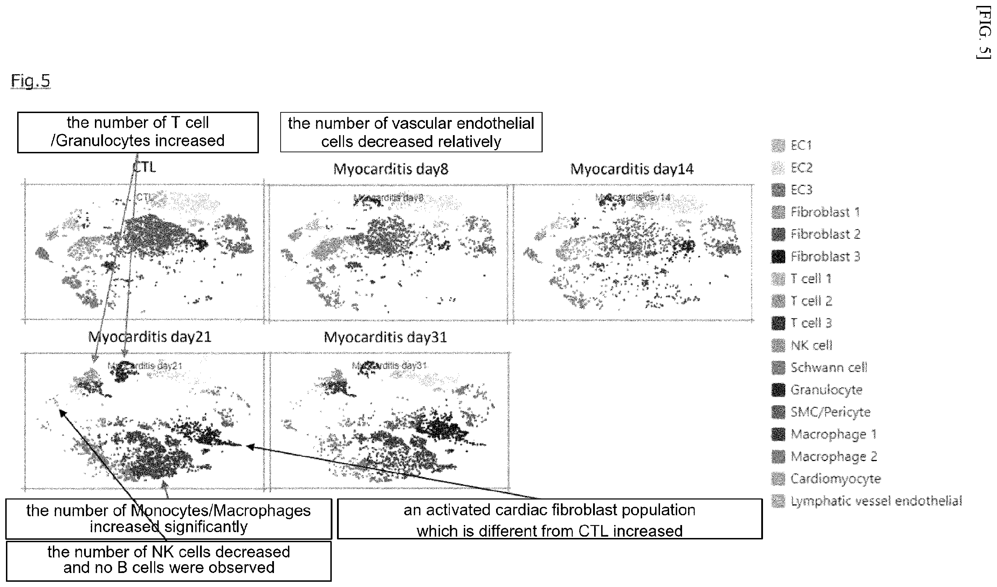

Single-cell analysis was performed by dispersing cells from a rat heart with an enzyme (Liberase TM (Roche, 5401119001/Liberase DH (Roche, 5401054001)) and analyzing them with Chromium (10x Genomics). FIG. 5 shows a result. In the figure, "CTL" denotes a control not administered with an antigen, and "Myocarditis day ..." denotes the number of days elapsed since the administration of the Pig Cardiac Myosin. On Day 21, the number of T cells and neutrophils increased, while the number of vascular endothelial cells decreased relatively. In addition, the number of monocytes and macrophages was also significantly increased, while the number of NK cells decreased and no B cells were observed. Also, no eosinophils were detected. Furthermore, unlike CTL, an activated cardiac fibroblast population increased.

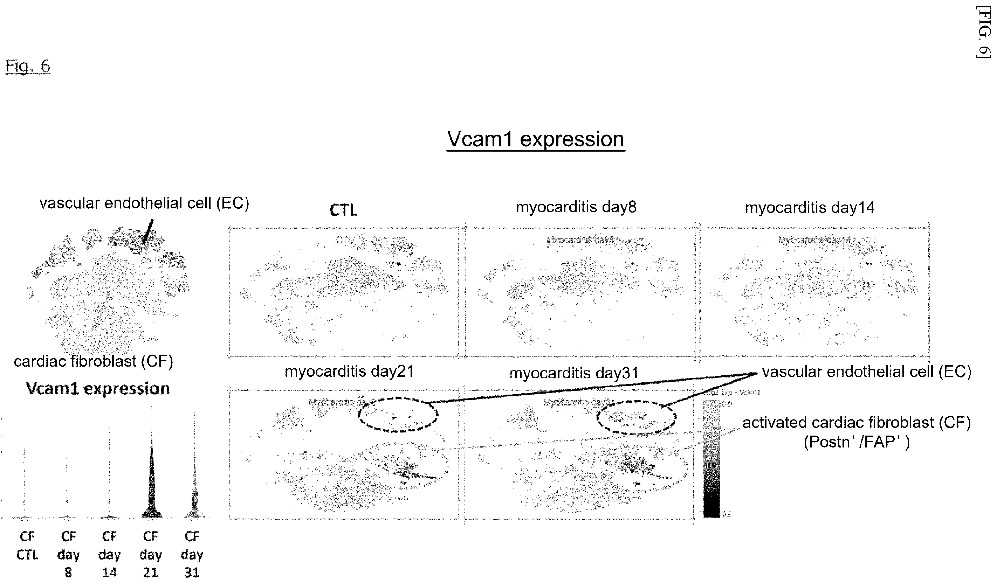

FIG. 6 shows expressions of Vcam-1 in the CTL and on day 8, day 14, day 21, and day 25. The Vcam-1 was expressed in the vascular endothelial cells, and was also highly expressed in the activated cardiac fibroblasts (Postn+/FAP+).

### 3. Adhesion experiment of monocytes with cardiac fibroblasts in vitro

This experiment was conducted to determine whether the expression of the Vcam-1 is altered by stimulation with TNFα, an inflammatory cytokine, and whether the adhesiveness between cardiac fibroblasts and monocytes is altered by the stimulation with the TNFα.

### (i) Culture and adhesion conditions

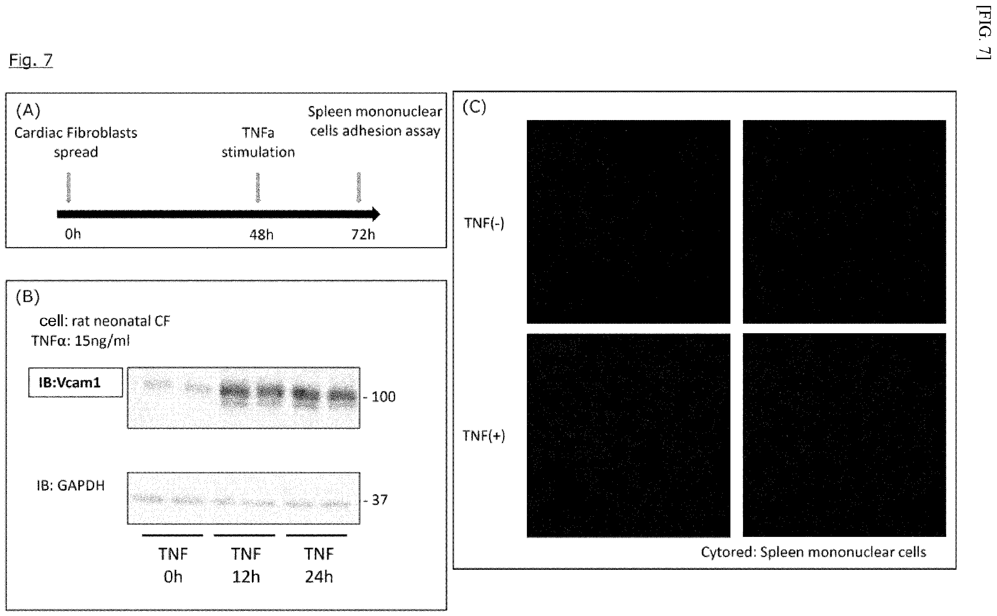

FIG. 7A shows a schedule of an adhesion experiment.

For the cardiac fibroblasts, fibroblasts isolated after cell dispersion of a heart harvested from a neonatal rat with Collagenase type 2 (Worthington Biochemical Corporation, LS004176) were cultured in a 96-well plate. For splenic monocytes, a spleen was harvested from a wild-type mouse and homogenized, and then monocyte fractions were collected by specific gravity separation using Optiprep (Serumwerk Bernburg, 1893). For the adhesion experiment, the cardiac fibroblasts were cultured in the 96-well plate for 48 hours, and then medium was replaced with TNFα +/- and cultured for another 24 hours. The splenic monocytes subjected to fluorescent staining (Hoechst 33342 (DOJINDO LABORATORIES, 341-07901) or Cytored (DOJINDO LABORATORIES, 342-08531)) were added and incubated at 37°C for 30 min. After washing 6 times with PBS(-) to remove non-adherent monocytes, adherent monocytes were counted using In Cell Analyzer 6000 (GE Healthcare Life Sciences).

The TNFα (Peprotech, 300-01A) was added to the wells at a final concentration of 5 to 15 ng/ml.

Induction of the Vcam-1 by the TNFα was determined by Western blotting. Anti-Vcaml antibody EPR5047 (Abcam, ab134047) was used as a primary antibody for the Western blotting. An HRP-conjugated anti-Rabbit antibody was used for detection, and Chemi-Lumi One L (Nacalai tesque, 07880-70) was used as a luminescence reagent. The Western blotting for GAPDH was performed as an internal control.

### (ii) Results

FIG. 7B shows results of the Western blotting. The Western blotting was performed in duplicates for each sample. The Vcam-1 was slightly expressed even at 0 hour after the addition of the TNFα. However, the expression of the Vcam-1 increased more at 12 hours and 24 hours after the addition of the TNFα. In addition, FIG. 7C is an image captured using the In Cell Analyzer 6000. As shown in FIG. 7C, it was shown that the adhesion between cardiac fibroblasts and monocytes was strengthened by a stimulation with TNFα.

### 4. Adhesion experiment between cardiac fibroblasts and monocytes using siRNA in vitro

To confirm direct adhesion of Vcam-1 to cardiac fibroblasts and monocytes, cardiac fibroblasts in which Vcam-1 was knocked down using siRNA were used to determine the adhesiveness between cardiac fibroblasts and monocytes.

### (i) Experimental conditions

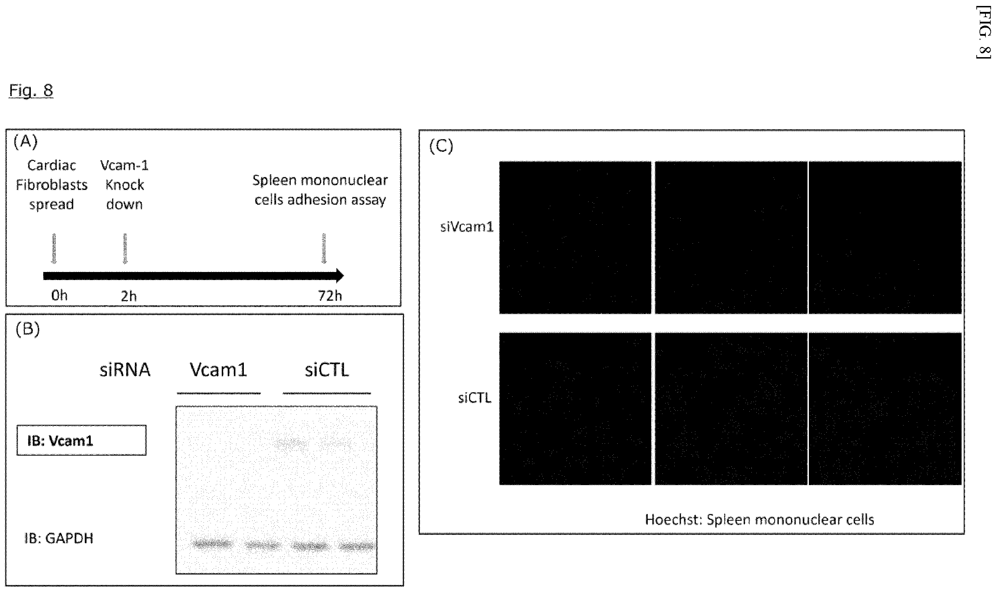

FIG. 8A shows a schedule of this experiment.

The culture of the cardiac fibroblasts and the adhesion experiment were performed as described in the above section 3 (i).

Vcam-1 siRNA (SilencerTM Select Pre-Designed siRNA, siRNA ID s129593, Thermo; also referred to as siVcam-1) was transfected into the cardiac fibroblasts at 2 hours after plating in wells using LipofectamineTM RNAiMAX Transfection Reagent (Thermo, 13778075) at a final concentration of 30 nM. The adhesion experiment with the monocytes was performed 72 hours after plating the cardiac fibroblasts into the wells. SilencerTM Select Negative Control No.1 siRNA (Thermo, 4390843) was used as a control for the siRNA (siCTL). The SilencerTM Select Negative Control No.1 siRNA was transfected at the same amount as the siVcam-1. In addition, the TNFα was added at 5 ng/ml at 46 hours after the transfection.

Western blotting for the Vcam-1 was performed as described in the above section 3 (i).

For detection of the monocytes, the adherent monocytes were counted using the In Cell Analyzer 6000 (GE Healthcare Life Sciences).

### (ii) Results

As shown in FIG. 8B, a signal of the Vcam-1 was detected in the cardiac fibroblasts transfected with the siCTL, whereas the signal of the Vcam-1 was not detected in the cardiac fibroblasts transfected with the siVcam-1. This indicates that siVcam-1 suppresses the expression of Vcam-1 in cardiac fibroblasts. This experimental system was used to determine the adhesiveness between the cardiac fibroblasts and the monocytes. FIG. 8C shows results. The adhesion of the monocytes was reduced in the cells transfected with the siVcam-1. On the other hand, the adhesion of the monocytes was observed in the cells transfected with the siCTL. This indicates that the adhesion between cardiac fibroblasts and monocytes is mediated by Vcam-1.

### 5. Verification of effect of combination of Vcam-1-neutralizing antibody and VLA-4-neutralizing antibody in improving prognosis of myocarditis

According to literature: Int Immunol. 2010 Jun; 22(6): 443-52, MRL-Pdcd1-/-, which is obtained by crossing MRL-Faslpr/lpr, a spontaneous systemic lupus erythematosus model mouse, with C57BL/6-Pdcd1-/-, a PD-1 knockout mouse, is a spontaneous lymphocytic myocarditis model in which 96% of the mice develop myocarditis at 4 to 8 weeks after birth. In addition, survival rate of the MRL-Pdcd1-/- mice at 10 weeks after birth is about 30%, which is significantly lower than that of wild-type mice. The MRL-Pdcd1-/- mice were used in the following experiments on myocarditis.

### (i) Combined administration of Vcam-1-neutralizing antibody and VLA-4-neutralizing antibody

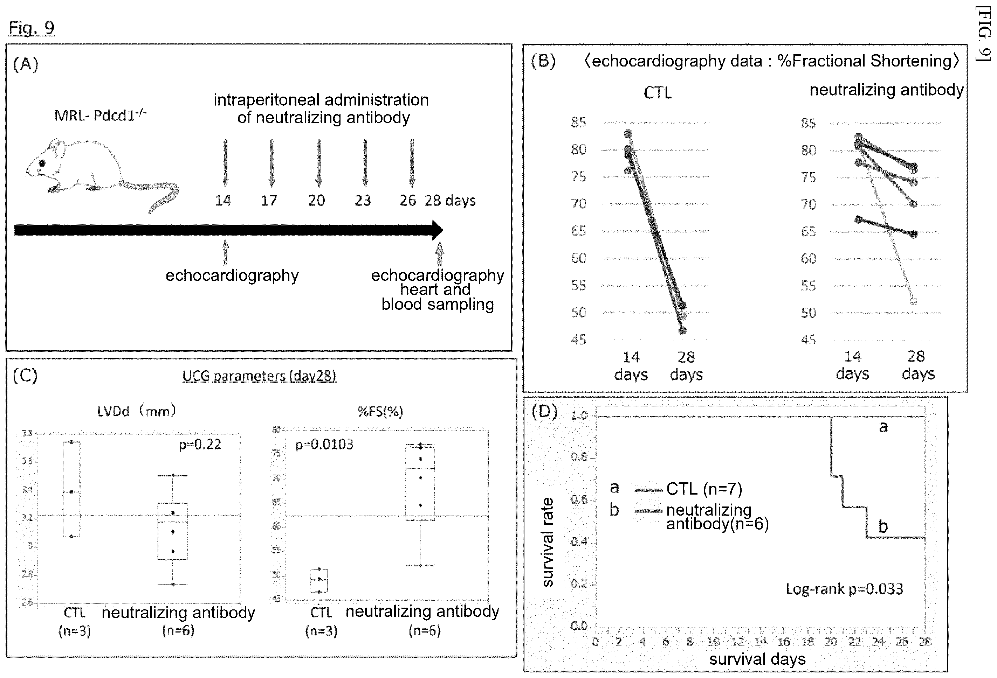

Thirteen MRL-Pdcd1-/- mice were divided into two groups: control (CTL: n=7) and a combined administration of Vcam-1-neutralizing antibody and VLA-4-neutralizing antibody (neutralizing antibody-administered group: n=7). From day 14 after birth, the CTL group was administered with saline intraperitoneally, and the neutralizing antibody-administered group was administered with the Vcam-1-neutralizing antibody and the VLA-4-neutralizing antibody. InVivoMAb anti-mouse CD106 (VCAM-1) (clone No. M/K-2.7, Cat No. BE0027, Bio X Cell) was used as the Vcam-1-neutralizing antibody. InVivoMAb anti-mouse/human VLA-4 (CD49d) (clone No. PS/2, Cat No. BE0071, Bio X Cell) was used as the VLA-4-neutralizing antibody. Each dose was 10 µg/g. For an administration schedule, as shown in FIG. 9A, the administration was performed every 3 days from day 14 to day 26 after birth. Echocardiography was performed on day 14 and day 28 after birth. After acquisition of echocardiography data on day 28 after birth, blood and hearts were collected from all mice.

### (ii) Results

FIG. 9B shows left ventricular fractional shortening (% fractional shortening; FS) measured by the echocardiography in all mice in the CTL group and the neutralizing antibody-administered group. In the CTL group, ventricular contractility was significantly reduced on day 28 after birth compared to that on day 14 after birth. On the other hand, the reduction of the ventricular contractility was milder in the neutralizing antibody-administered group than in the CTL group. FIG. 9C shows boxplots of left ventricular end-diastolic diameter [LVDd (mm)] and the left ventricular fractional shortening [%FS (%)] on day 28 in the same mice as in FIG. 9B. There was no significant difference in the LVDd between the CTL group and the neutralizing antibody-administered group (p=0.22). On the other hand, the %FS was significantly higher in the neutralizing antibody-administered group (p=0.0103). FIG. 9D shows Kaplan-Meier curves for the CTL group and the neutralizing antibody-administered group. In the figure, sign "a" denotes the CTL group and sign "b" denotes the neutralizing antibody-administered group. In an examination up to day 28 after birth, the number of mice in the CTL group that died increased from day 20 after birth. On the other hand, no mice in the neutralizing antibody-administered group died. A significant difference between the two groups was shown by a Log-rank test (p=0.033). This indicates that a combined administration of Vcam-1-neutralizing antibody and VLA-4-neutralizing antibody ameliorates mortality due to myocarditis.

### 6. Effect of VLA-4-neutralizing antibody

The following examination was performed to determine whether Vcam-1-neutralizing antibody or VLA-4-neutralizing antibody contribute to lowering the mortality due to myocarditis.

### (i) Administration of antibody

Twenty-nine MRL-Pdcd1-/- mice were divided into four groups: control (CTL: n=12), Vcam-1 group (n=5) to be administered with only Vcam-1-neutralizing antibody, VLA-4 group (n=6) to be administered with only VLA-4-neutralizing antibody, and Vcam-1/VLA-4 group (n=6) to be administered with a combination of Veam-1-neutralizing antibody and VLA-4 neutralizing antibody.

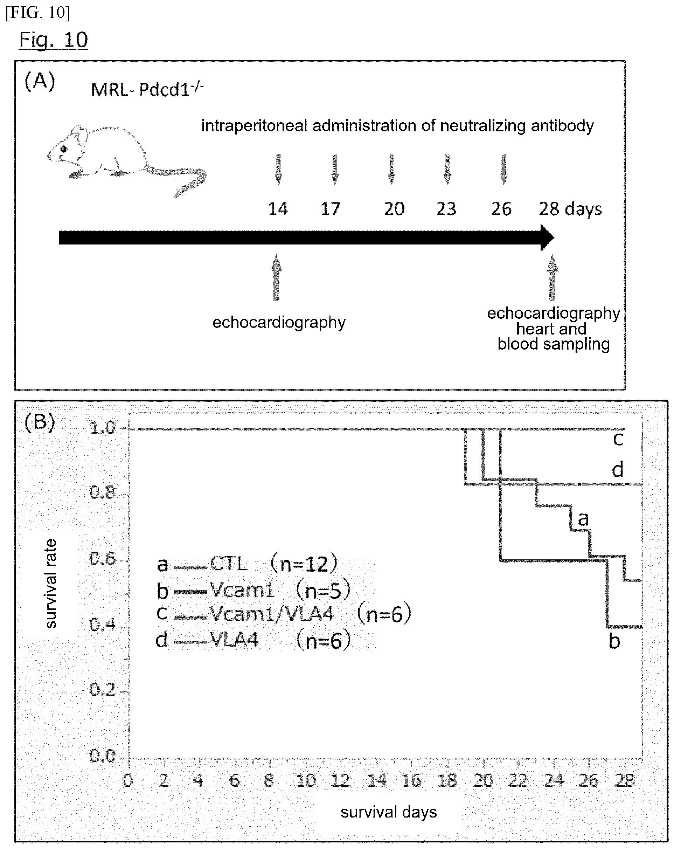

The dose of each antibody was the same as in the above section 5 (i). As shown in FIG. 10A, an administration schedule of the neutralizing antibody was also the same as in the above section 5 (i).

### (ii) Results

FIG. 10B shows Kaplan-Meier curves for each group. In the figure, sign "a" denotes the CTL group, sign "b" denotes the Vcam-1 group, sign "c" denotes the Vcam-1/VLA-4 group, and sign "d" denotes the VLA-4 group. In an examination up to day 28 after birth, the number of mice in the CTL group (sign "a") that died increased from day 19 after birth. The number of mice in the Vcam-1 group (sign "b") that died also increased from day 20 after birth. On the other hand, no mice in the Vcam-1/VLA-4 group (sign "c") died. In the VLA-4 group (sign "d"), one mouse died on day 18 after birth, but the remaining mice survived until day 28 after birth. This indicates that it is VLA-4-neutralizing antibody that contributes to improving the prognosis of myocarditis.

### 7. Effect of VLA-4-neutralizing antibody in group of mice with reduced cardiac function

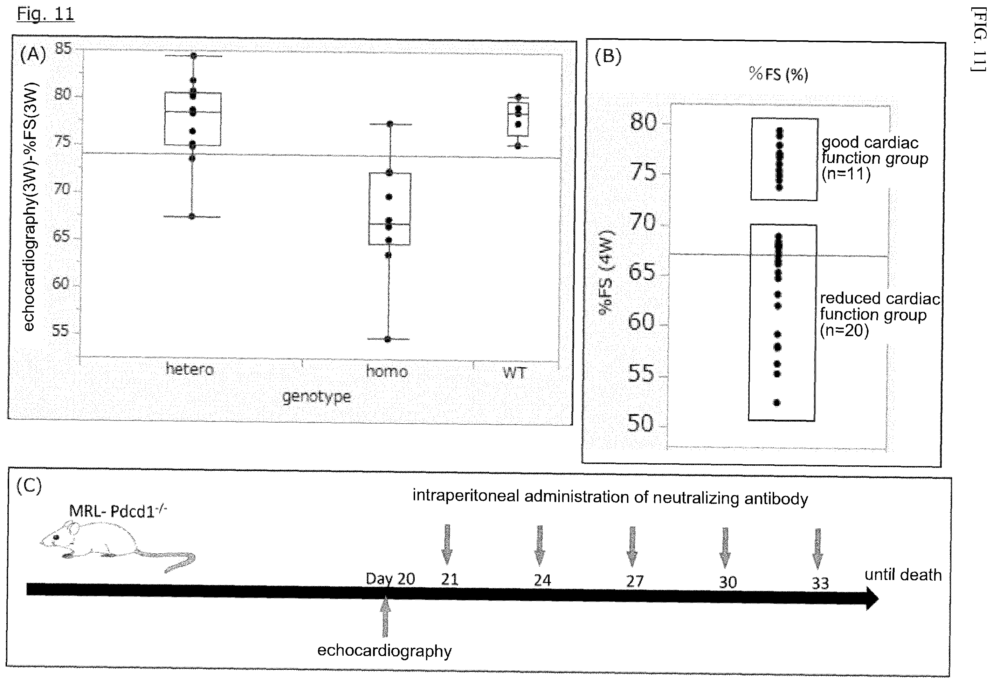

FIG. 11A shows the %FS values of wild-type mice (WT), F1 mice (hetero: MRL-Pdcd1-) obtained by crossing wild-type mice with MRL-Pdcd1-/- mice, and MRL-Pdcd1-/- mice (homo) at 3 weeks after birth. The %FS value of the WT group was 75% or more, with little difference between the first and fourth quartiles, indicating that the variation was small. On the other hand, the %FS values of the homo group and the hetero group were broadly distributed as a whole, with some individuals having high %FS values and others having low %FS values.

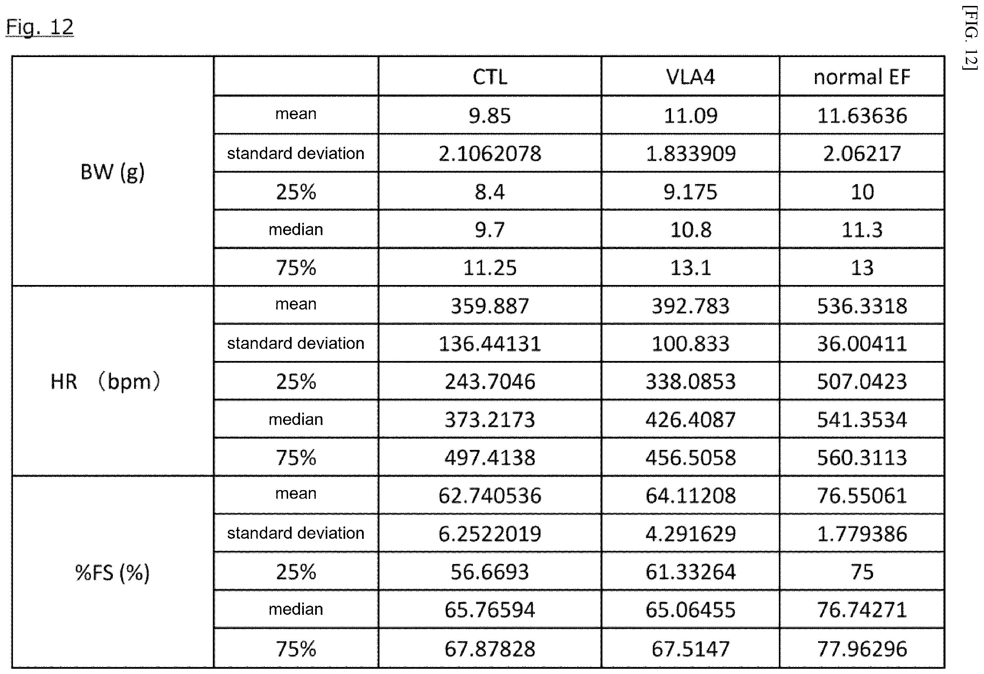

Accordingly, as shown in FIG. 11B, the homo group was divided into a high %FS group (good cardiac function group: n=11) and a low %FS group (reduced cardiac function group: n=20), and the reduced cardiac function group was further divided into a control group not to be administered with the VLA-4-neutralizing antibody (CTL group) and a VLA-4-administered group to be administered with the VLA-4-neutralizing antibody (VLA-4 group), and the effect of the VLA-4-neutralizing antibody was determined. A cutoff value to distinguish between the good cardiac function group (normal EF group) and the reduced cardiac function group was set to 70 %, and the group with 70 % or more was defined as the good cardiac function group, while the group with less than 70 % was defined as the reduced cardiac function group. Then, the VLA-4-neutralizing antibody was administered according to a schedule shown in FIG. 11C. FIG. 12 shows body weights (BW), pulse rates (HR), and the % FS values for each group. There was no significant difference in each parameter between the CTL group and the VLA-4 group.

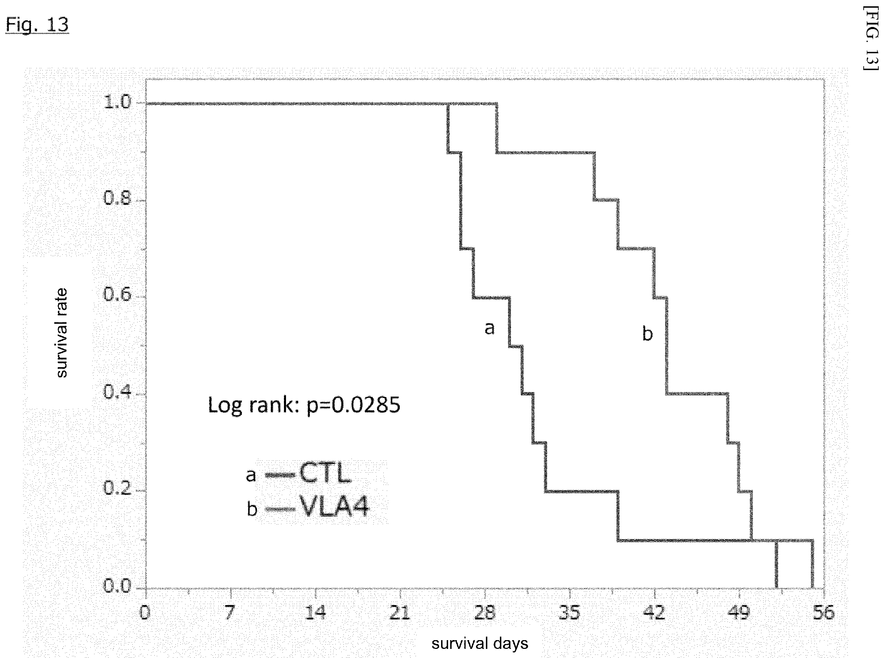

FIG. 13 shows Kaplan-Meier curves for the CTL group (sign "a") and the VLA-4 group (sign "b"). As a result of the Log-rank test, p=0.0285 was obtained, and survival rate was significantly prolonged in the VLA-4 group.

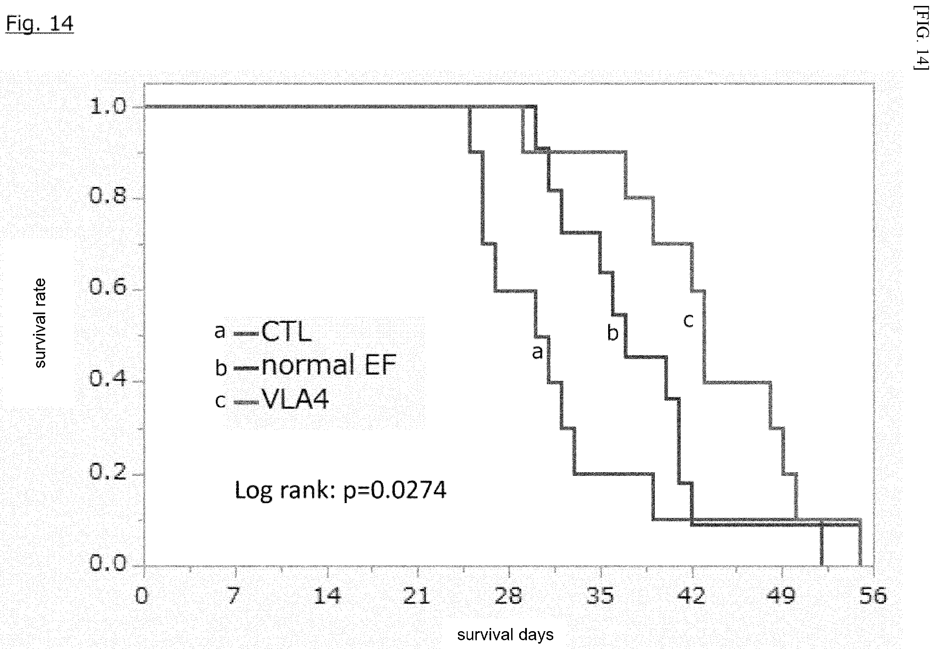

FIG. 14 shows the Kaplan-Meier curves with the addition of a survival curve for the normal EF group (sign "b"). The normal EF group, which had good cardiac function and was not administered with the VLA-4-neutralizing antibody, showed a significantly lower survival rate than the VLA-4 group (as a result of Log-rank test, p=0.0275).

This suggests that administration of VLA-4-neutralizing antibody to individuals with myocarditis improves the prognosis of myocarditis independent of cardiac function data of each individual. In addition, it is suggested that administration of VLA-4-neutralizing antibody to individuals with myocarditis is effective in preventing or treating myocarditis.

### 8. Effect of administration of VLA4 inhibitor

The following examination was performed to determine whether administration of AJM300 (carotegrast methyl), one of VLA4 inhibitors, contributes to lowering mortality due to myocarditis.

### (i) Administration of AJM300

Forty-four MRL-Pdcd1-/- mice were divided into two groups: AJM300(-) group (n=13) not to be administered with AJM300 and AJM300(+) group (n=12) to be administered with AJM300.

The AJM300 group (+) was administered with a mixed diet containing 1% of AJM300 from 2 weeks of age until death.

At 28 days of age, 9 mice in the AJM300(-) group and 10 mice in the AJM300(+) group were evaluated for cardiac function by echocardiography and then euthanized to collect heart and blood samples.

### (ii) Results

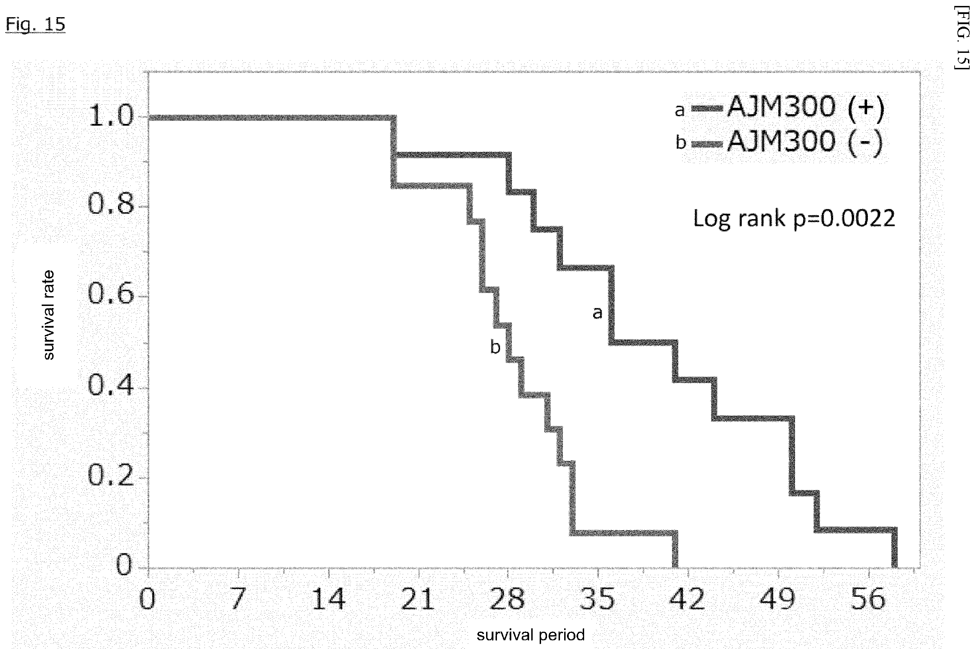

FIG. 15 shows Kaplan-Meier curves for each group. In the figure, the sign "a" denotes the AJM300(+) group, and the sign "b" denotes the AJM300(-) group. Survival period was significantly prolonged in the AJM300(+) group compared with the AJM300(-) group (Log-rank test, p=0.0022). This indicates that administration of a VLA4 inhibitor contributes to improving the prognosis of myocarditis.

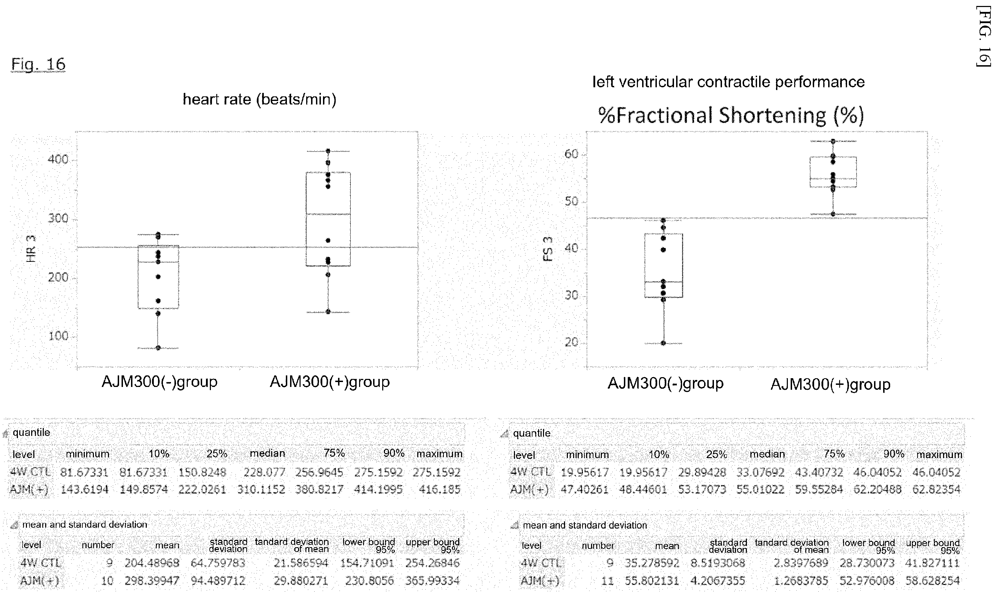

FIG. 16 shows results of heart rate and left ventricular contractile performance: % Fractional Shortening (%) as measured by cardiac function test by the echocardiography. The AJM300(+) group had significantly ameliorated bradycardia compared to the AJM300(-) group (t-test, p=0.0231). In addition, the AJM300(+) group had significantly improved left ventricular contractile performance compared to the AJM300(-) group (t-test, p=0.0231). This indicates that administration of an inhibitor of VLA4 contributes to improving cardiac function.

### 9. Level of soluble Vcam-1 in serum in humans

Levels of soluble Vcam-1 (sVcam-1) in serum were compared between healthy subjects and patients with myocarditis.

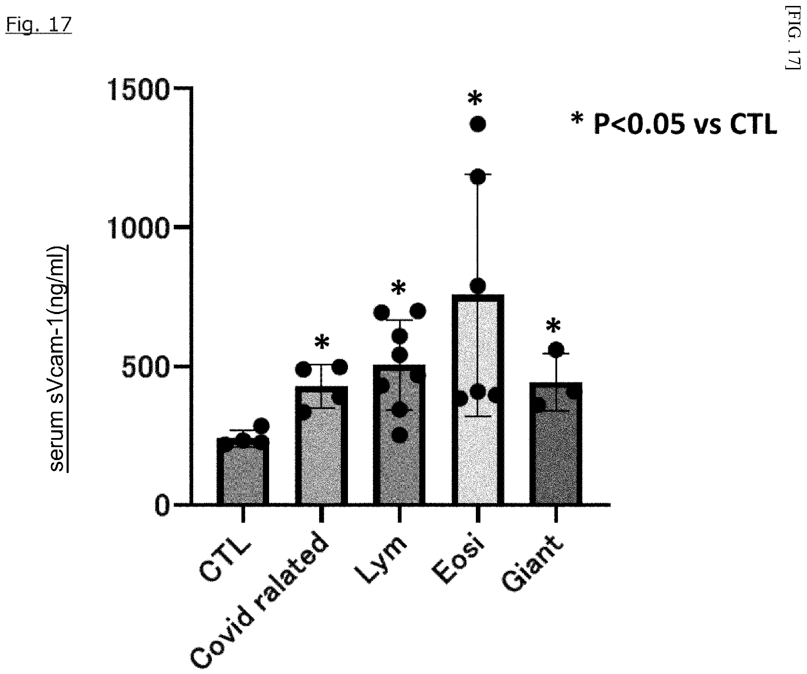

FIG. 17 shows levels of soluble Vcam-1 in sera of three healthy subjects (CTL), four patients with Covid-related myocarditis (Covid related), eight patients with lymphocytic myocarditis (Lym), six patients with eosinophilic myocarditis (Eosi), and three patients with giant cell myocarditis (Giant). In all types of myocarditis, including the lymphocytic myocarditis, the serum sVcam-1 levels increased compared to that in the healthy subjects. This suggests that a VLA-4/Vcam-1 pathway is involved in onset of myocarditis.

### 10. Transition of serum sVcam-1 in myocarditis pathology

The serum sVcam-1 levels in the three patients with lymphocytic myocarditis were compared in acute and convalescent phases.

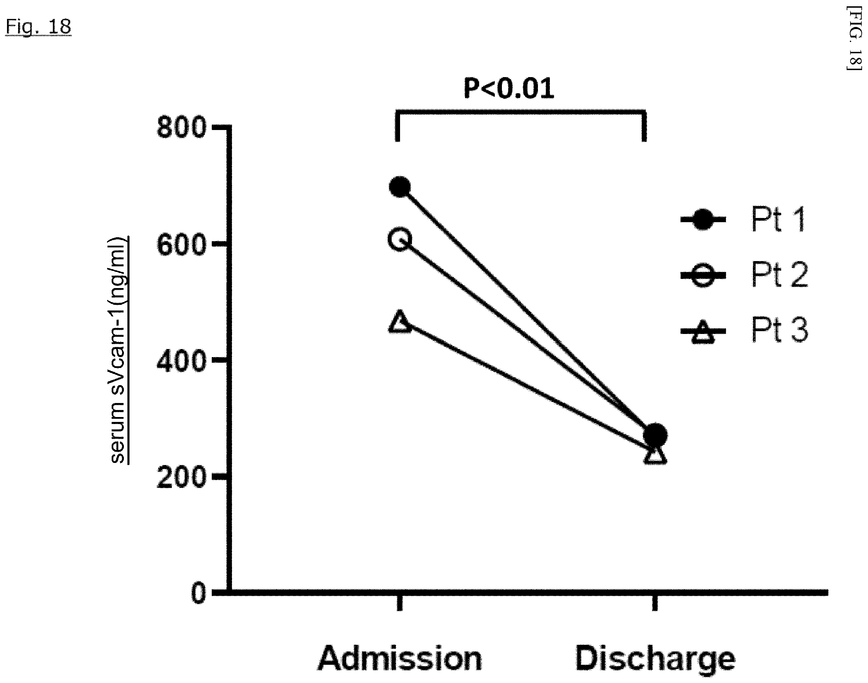

FIG. 18 shows results. The serum sVcam-1 levels of the three patients were very high in the acute phase. As can be seen from the comparison with the serum sVcam-1 levels of healthy subjects shown in FIG. 17, the levels of sVcam-1 of the three patients also improved to the level of the healthy subjects in the convalescent phase.

This suggests that the VLA-4/Vcam-1 pathway is involved in myocarditis.

### 11. Evaluation of [¹¹C]HCA2969 binding to VLA-4 in mouse monocytes

Monocytes collected from a mouse were used to evaluate the binding of a [¹¹C]HCA2969 to VLA-4.

### (i) Methods

2 × 10⁶ monocytes collected from a spleen of a mouse and a [¹¹C]HCA2969 synthesized in the above section 1 were added to buffer (10mM Tris pH 7.5, 0.1% BSA, 150 mM NaCl, 2 mM MnCl₂, 2 mM D-glucose) at concentrations of 0.025 nM, 0.1 nM, 0.4 nM, 1.6 nM, 6.4 nM and 25.6 nM, respectively, and mixed. After mixing, the cells were incubated at room temperature for 20 minutes and washed 2 times with buffer (10mM Tris pH 7.5, 0.1% BSA, 150 mM NaCl, 2 mM MnCl₂, 2 mM D-glucose). Radiation dose was measured for the washed cells using a scintillation counter.

### (ii) Results

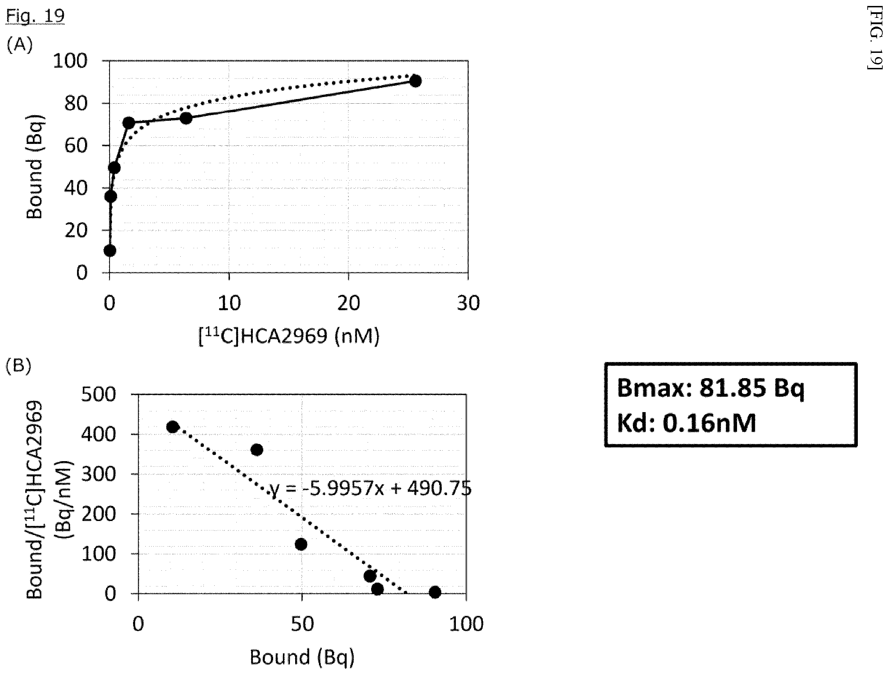

FIG. 19A shows the radiation dose at each addition amount of the [¹¹C]HCA2969. FIG. 19B shows the radiation dose per 1 nM of the [¹¹C]HCA2969. A clear saturation curve was obtained in FIG. 19A, and an equilibrium dissociation constant Kd of 0.16 nM and a Bmax of 81.85 Bq were calculated by Scatchard analysis in FIG. 19B.

### 12. Detection of myocarditis using [¹¹C]HCA2969

In the MRL-Pdcd1-/- and wild-type mice prepared in the above section 5, a [¹¹C]HCA2969 tracer (about 1 to 10 MBq) was administered intravenously via a tail vein under inhalation anesthesia with isoflurane on days 25 to 26, followed by PET/CT imaging.

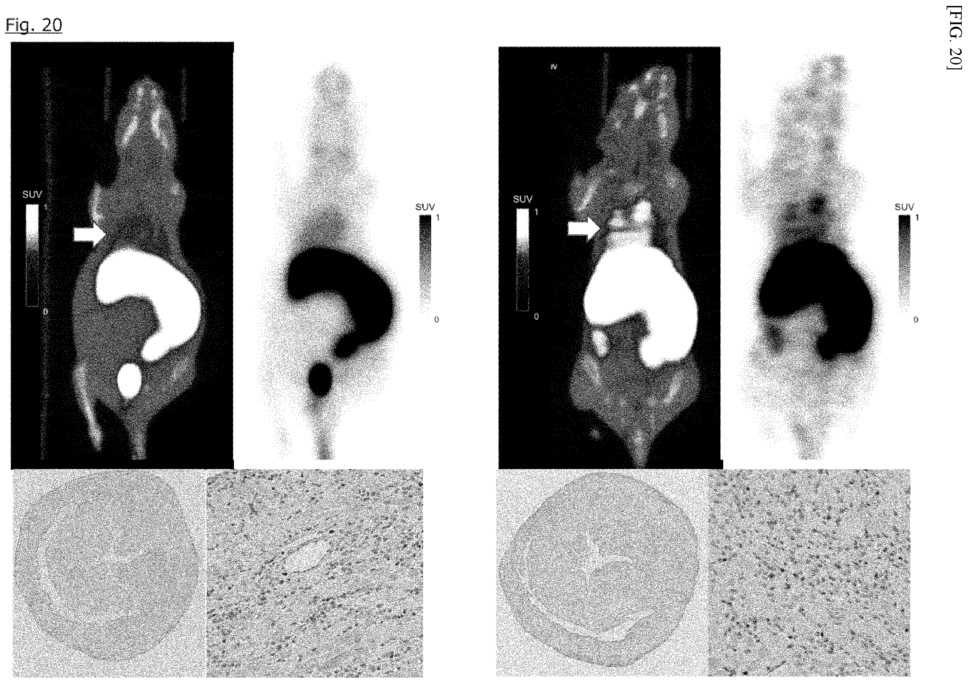

FIG. 20 shows results for two MRL-Pdcd1-/- mice. In both cases, tracer accumulation in their hearts was observed.

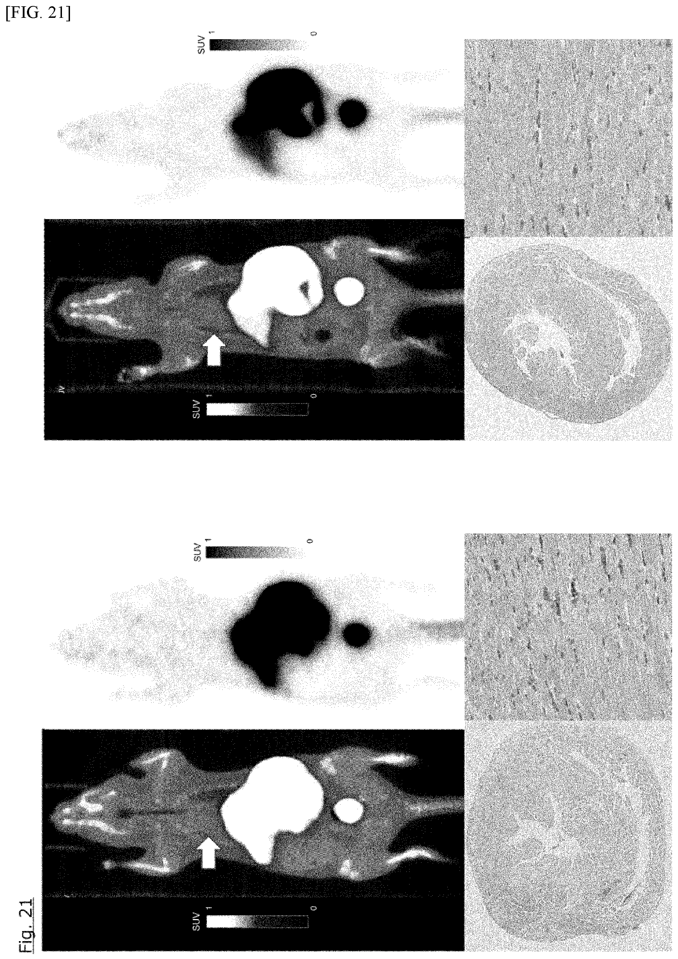

FIG. 21 shows results for two wild-type mice. In both cases, no tracer accumulation in their hearts was observed.

### 13. Detection of autoimmune myocarditis using [¹¹C]HCA2969

In autoimmune myocarditis model rat prepared in the above section 2, a [¹¹C]HCA2969 tracer at about 1 to 10 MBq was administered intravenously via a tail vein under inhalation anesthesia with isoflurane at 3 weeks of induction, which is the climax of myocarditis, followed by PET/CT imaging.

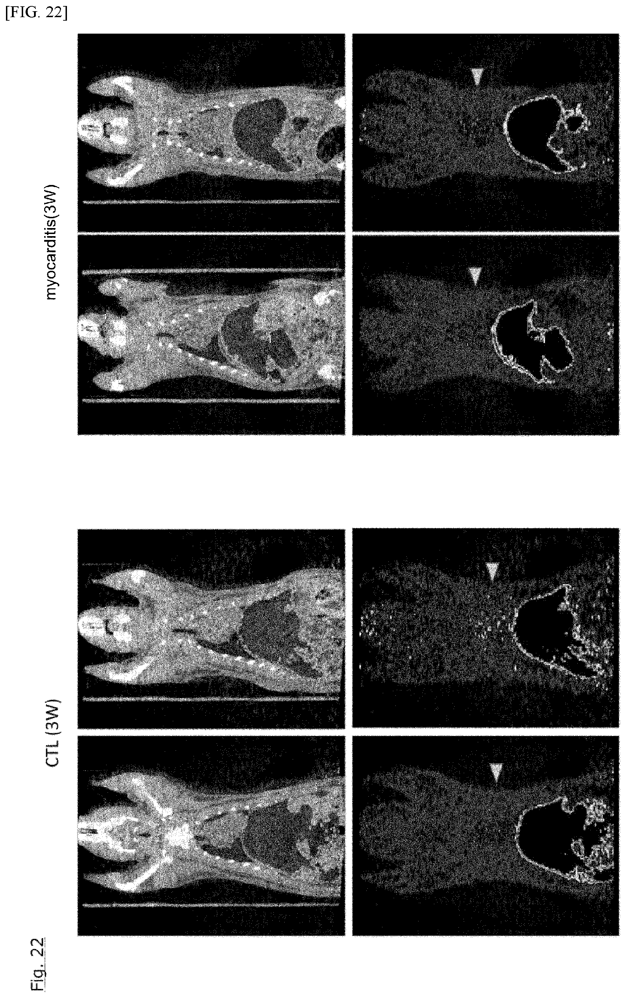

As shown in FIG. 22, no tracer accumulation in hearts was observed in autoimmune the myocarditis model rats (right: labeled "myocarditis") as well as in non-induced myocarditis rats (left: labeled "CTL").

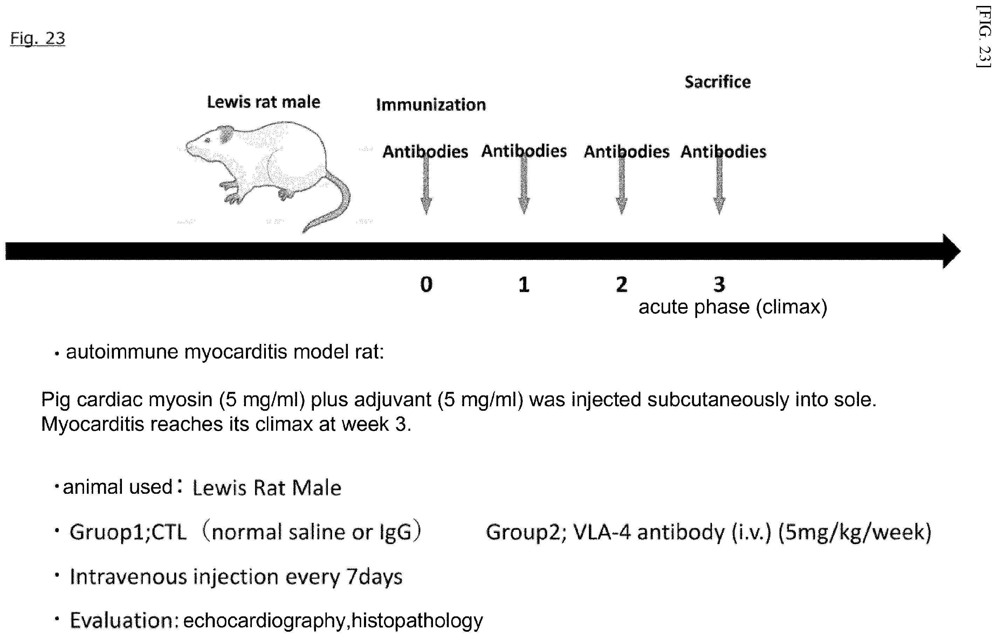

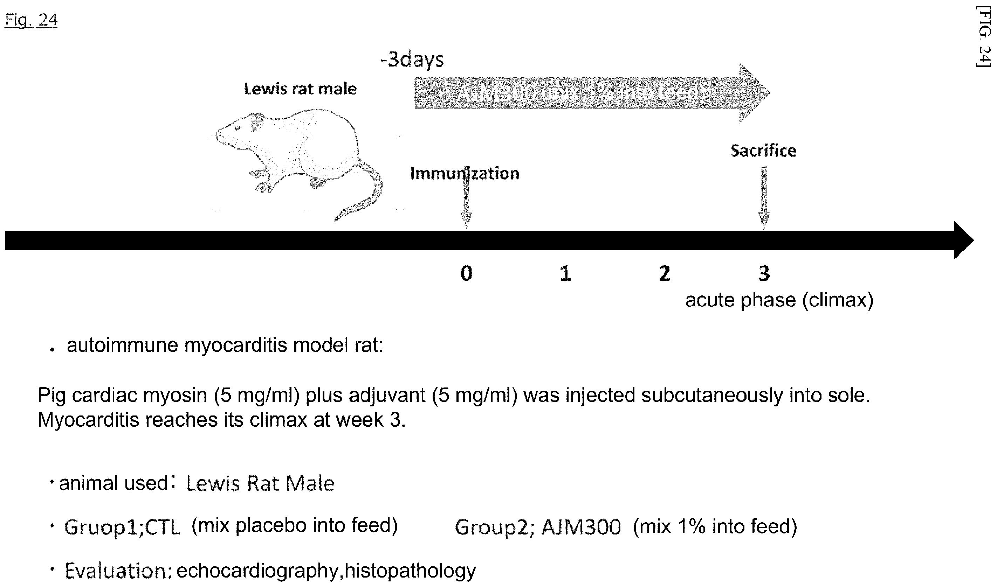

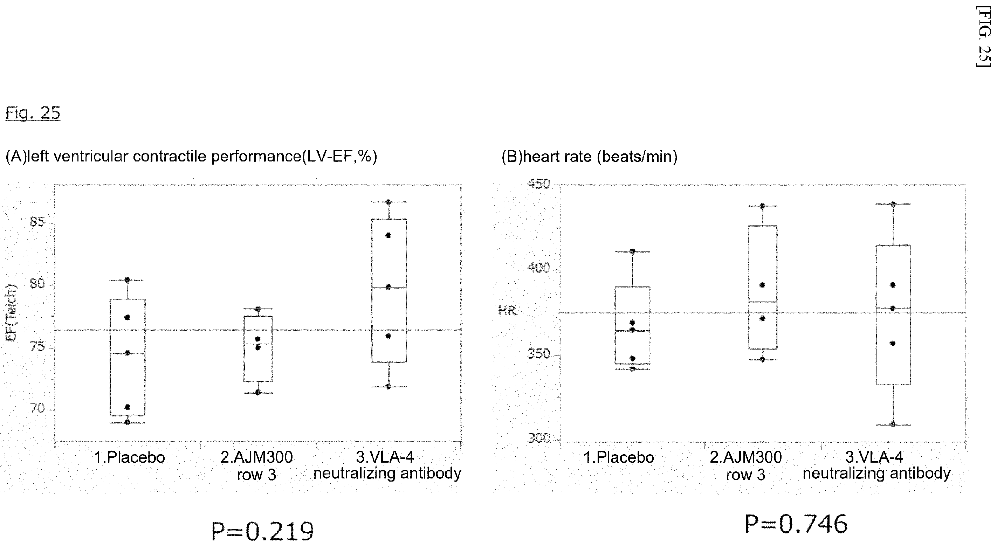

As shown in FIGS. 23 and 24, the anti-VLA-4 neutralizing antibody described above or AJM300 was administered to autoimmune myocarditis model rats. Study groups were: placebo group (5 animals), AJM300-administered group (4 animals), and neutralizing antibody-administered group (5 animals) administered with anti-VLA-4-neutralizing antibody. The placebo group was fed a diet mixed with placebo (0.43% METOLOSE + 0.14% Ac-Di-Sol) from 3 days before induction of myocarditis to 3 weeks. The AJM300-administered group was fed a diet mixed with 1% AJM300 from 3 days before induction of myocarditis to 3 weeks. The neutralizing antibody-administered group was intravenously administered with 5 mg/kg of neutralizing antibody at the time of induction of myocarditis (0w), 1 week after induction of myocarditis (1w), 2 weeks after induction of myocarditis (2w), and 3 weeks after induction of myocarditis (3w). Left ventricular contractile performance and heart rate were evaluated 21 days after induction of myocarditis. FIG. 25A shows left ventricular contractile performance in the placebo group, the AJM300-administered group, and the neutralizing antibody-administered group. FIG. 25B shows distribution of heart rate in the placebo group, the AJM300-administered group, and the neutralizing antibody-administered group. As shown in FIG. 25, in the autoimmune myocarditis model rats, there was no significant difference in cardiac function among the placebo group, the AJM300-administered group, and the neutralizing antibody-administered group, showing that AJM300 and anti-VLA-4 neutralizing antibody had no effect on improving cardiac function.

Combined with the results of section 12 above, it was demonstrated that the [¹¹C]HCA2969 tracer accumulates in myocardium in a myocarditis model in which therapeutic effects with AJM300 and anti-VLA-4 neutralizing antibodies can be expected.

### IV. Determination of therapeutic effect using [¹¹C]HCA2969

Next, in PD1 knockout mice (MRL-Pdcd1-/- mice: PD1-/- mice), the therapeutic effect on myocarditis was evaluated using a [¹¹C]HCA2969 tracer.

### (i) Methods

Groups of wild-type mice, placebo PD1-/- mice, AJM300-administered PD1-/- mice and anti-VLA-4 neutralizing antibody-administered PD 1-/- mice at 4 weeks of age were prepared. The placebo group was fed a normal diet and the AJM300-administered group was fed a diet containing 1% AJM300 from day 14 to day 28 after the start of rearing. The anti-VLA-4 neutralizing antibody-administered group was fed a normal diet and administered with anti-VLA -4 neutralizing antibody (10 mg/kg) intraperitoneally on days 14 and 21 after the start of rearing.

On day 28 after the start of rearing, each mouse was anesthetized with isoflurane (1 to 3% in 2 L of O₂) and injected with a [¹¹C]HCA2969 tracer (1 to 10 MBq: 10 µg of unlabeled HCA2969 containing 10 MBq of PET tracer) via a tail vein. Twenty minutes after the injection, PET/CT imaging was performed for 10 minutes.

### (ii) Results

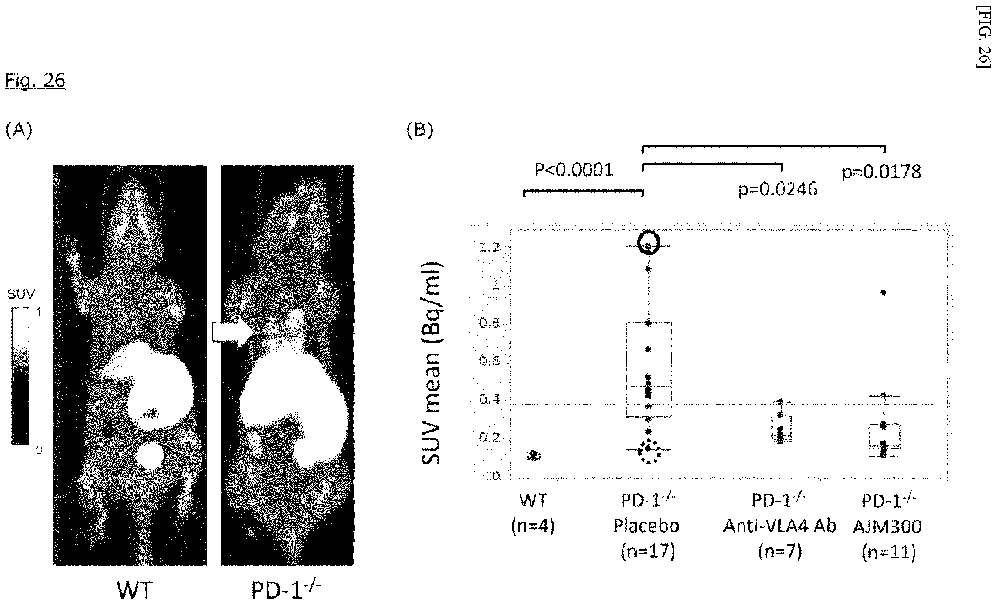

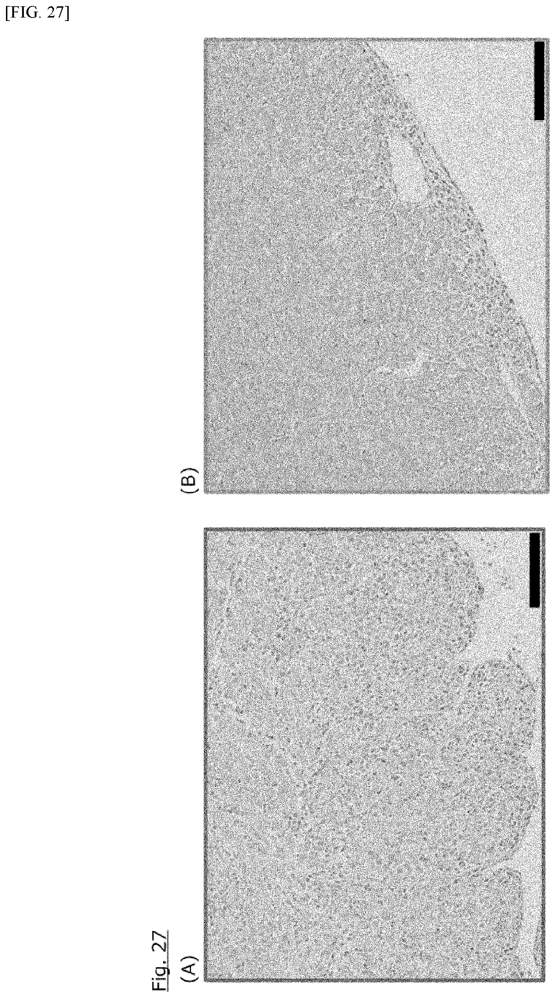

Results are shown in FIGS. 26 and 27. FIG. 26A shows an example of PET/CT imaging of a wild-type mouse (WT) and a PD1-/- mouse (PD1-/-). In the PD1-/- mouse, accumulation of the [¹¹C]HCA2969 tracer was observed in the cardiac region. FIG. 26B is a boxplot quantifying the accumulation in the cardiac region for each group.

In the placebo group of PD1-/- mice, accumulation of the [¹¹C]HCA2969 tracer was observed. On the other hand, accumulation of the [¹¹C]HCA2969 tracer was lower in the AJM300-administered group and the anti-VLA-4 neutralizing antibody-administered group compared to the placebo group.

These results suggest that the [¹¹C]HCA2969 tracer can evaluate the therapeutic effects of AJM300 treatment and anti-VLA-4 neutralizing antibody treatment.

FIG. 27A shows an image of myocardial tissue of an individual marked with a solid circle in the boxplot of the placebo group in FIG. 26B. This individual was the one with particularly high accumulation of the [¹¹C]HCA2969 tracer. FIG. 27B shows an image of myocardial tissue of an individual marked with a dotted circle in the boxplot of the placebo group in FIG. 26B. This individual was the one with particularly low accumulation of the [¹¹C]HCA2969 tracer. In FIG. 27A, monocyte infiltration into the tissue was observed, indicating severe inflammation of the myocardium. On the other hand, no inflammatory findings were observed in FIG. 27B. These results suggest that the [¹¹C]HCA2969 tracer specifically accumulates in inflamed myocardial tissue.

### 15. Diagnosis of VLA-4-positive lymphocyte-associated diseases other than myocarditis using [¹¹C]HCA2969 tracer

An Experimental Autoimmune Encephalitis (EAE) model (mouse encephalomyelitis model: EAE mice) was used to evaluate the feasibility of using [¹¹C]HCA2969 in diagnosis of VLA-4-positive lymphocyte-associated diseases other than myocarditis.

### 15-1. Observation of [¹¹C]HCA2969 tracer signal accumulation in affected area

### (i) Preparation of EAE mice (immunization)

Complete Freund's Adjuvant (CFA) was prepared by suspending 40 mg of H37RA Mycobacterium tuberculosis toxin (BD) in 10 ml of Adjuvant incomplete Freund (BD). MOG peptide (MOG 35-55, Biosynthesis) was dissolved in physiological saline to a concentration of 1 mg/ml. For each 9-week-old male C57BL/6J mouse, an emulsion solution was prepared by mixing and suspending 100 µl of CFA and 100 µl of MOG solution in a glass syringe. The resulting turbid emulsion solution was injected subcutaneously into both inguinal regions at a volume of 200 µl per mouse (day 0 of immunization). Subsequently, a solution of Pertussis toxin (LBL) dissolved in PBS (4 µg / ml) was administered intraperitoneally at 100 µl per mouse on the day of administration of the emulsion solution, followed by a second intraperitoneal administration 48 hours later. Disease scores were recorded for EAE starting from day 0 of immunization.

In this model, infiltration of inflammatory cell into a spinal cord and paralysis symptoms appear around one week, peak at two weeks, and show improvement by four weeks post-immunization. Inflammation predominates in spinal cord over brain and occurs from lumbar spinal cord side, so paralysis begins in tail and hind limbs.

In the following experiments, mice exhibiting paralysis symptoms with an EAE disease score of 0.5 or higher at 15 days post-immunization were used.

### (ii) PET/CT imaging

On day 15 post-immunization, each mouse was anesthetized with isoflurane (1 to 3% in 2 L of O₂) and injected with a [¹¹C]HCA2969 tracer (1 to 10 MBq: 10 µg of unlabeled HCA2969 containing 10 MBq of PET tracer) via a tail vein. Twenty minutes after the injection, PET/CT imaging was performed for 10 minutes. For imaging, a region of interest (ROI) was set from thoracic spinal cord to lumbar spinal cord, accumulation of [¹¹C]HCA2969 tracer signal was counted, and SUV median (Bq / ml) and SUV mean (Bq / ml) were calculated.

### (iii) Results

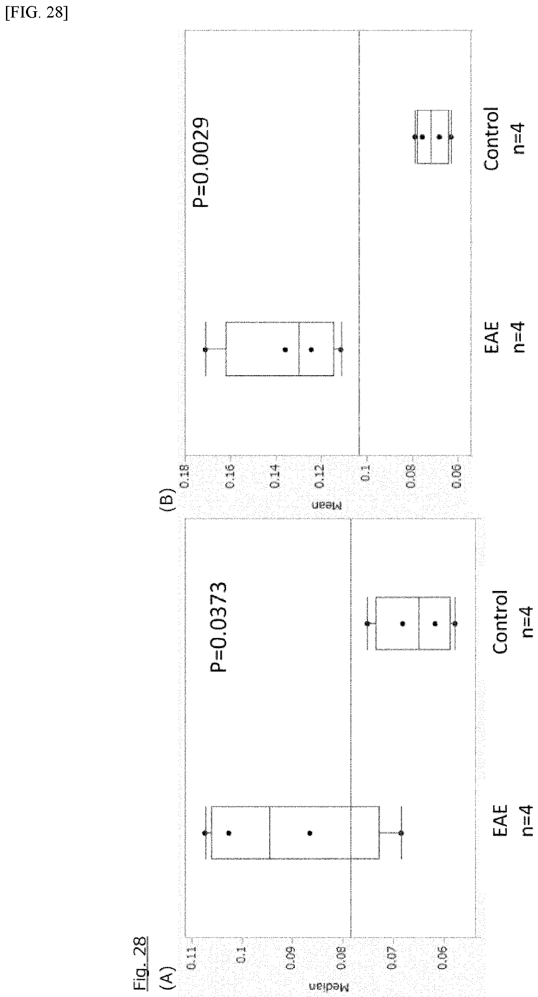

FIG. 28 shows SUV median (Bq/ml) and SUV mean (Bq/ml) for a negative control group (Control) and an EAE model group (EAE). FIG. 28A shows the SUV median (Bq/ml), and FIG. 28B shows the SUV mean (Bq/ml). In both cases, high accumulation of the [¹¹C]HCA2969 tracer signal was observed in the EAE model group, indicating that the [¹¹C]HCA2969 tracer of the present invention can detect inflammation in tissues other than heart.

### 15-2. Observation of [¹¹C]HCA2969 tracer uptake into affected area

Uptake of a [¹¹C]HCA2969 tracer into spinal cord and brain was observed using the EAE model mice.

### (i) Methods

In the EAE model prepared in the above section 17-1, a [¹¹C] HCA2969 tracer was administered to mice presenting paralysis symptoms with an EAE disease score of 0.5 or higher on day 15 post-immunization according to the method described in the above section 17-1 (ii). Thirty minutes after administration, the mice were euthanized, and spinal cords and brains were harvested. A portion of the harvested tissue was used to measure the radiation dose using a Well counter, and another portion was subjected to HE staining.

### (ii) Results

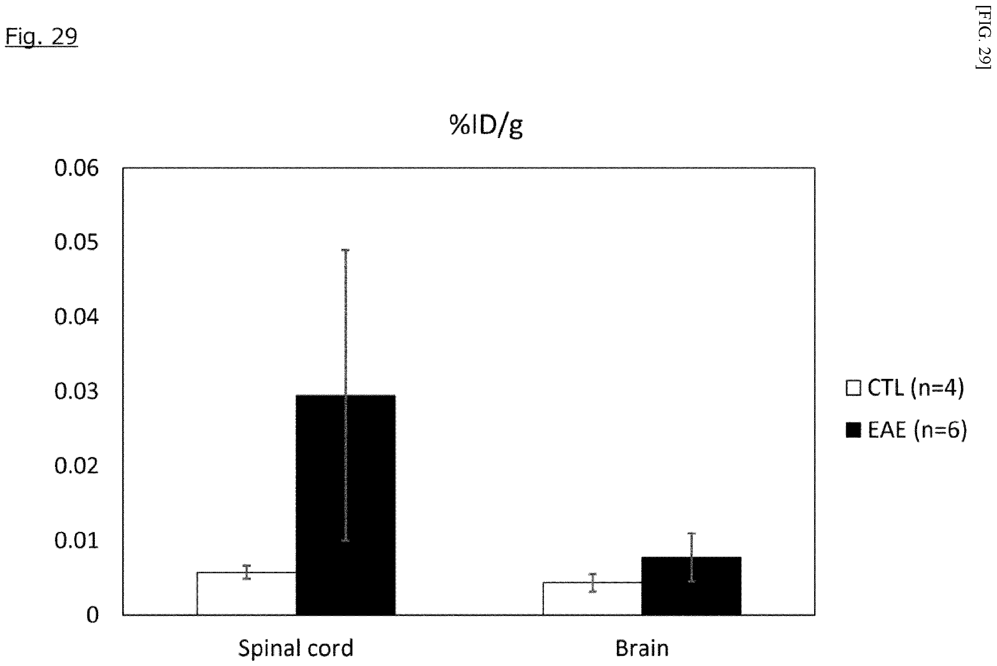

FIG. 29 shows measurement results of the well counter. Compared to the negative control group (CTL group), the EAE model group exhibited a significant increase in accumulation of the tracer signal in the spinal cord. In the brain, an increasing trend in accumulation was also observed. Since infiltration of inflammatory cells occurs primarily in the spinal cord in the EAE model, this accumulation was considered to reflect infiltration of inflammatory cells.

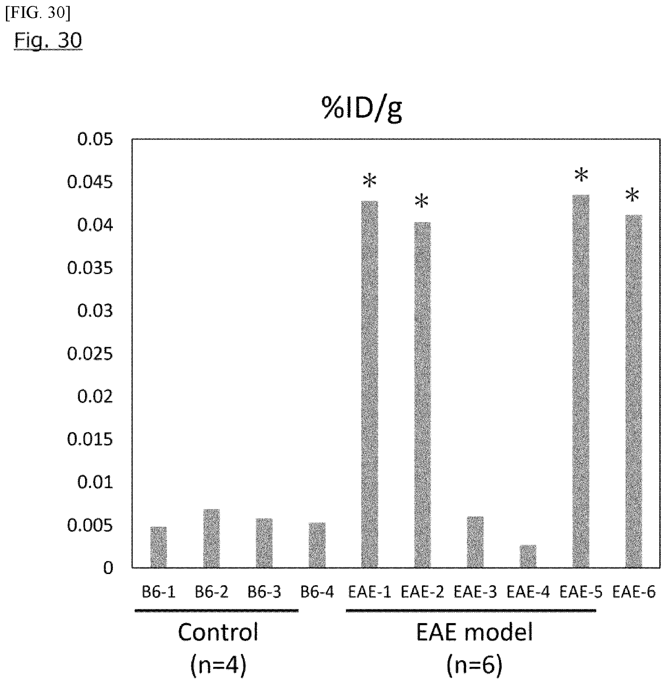

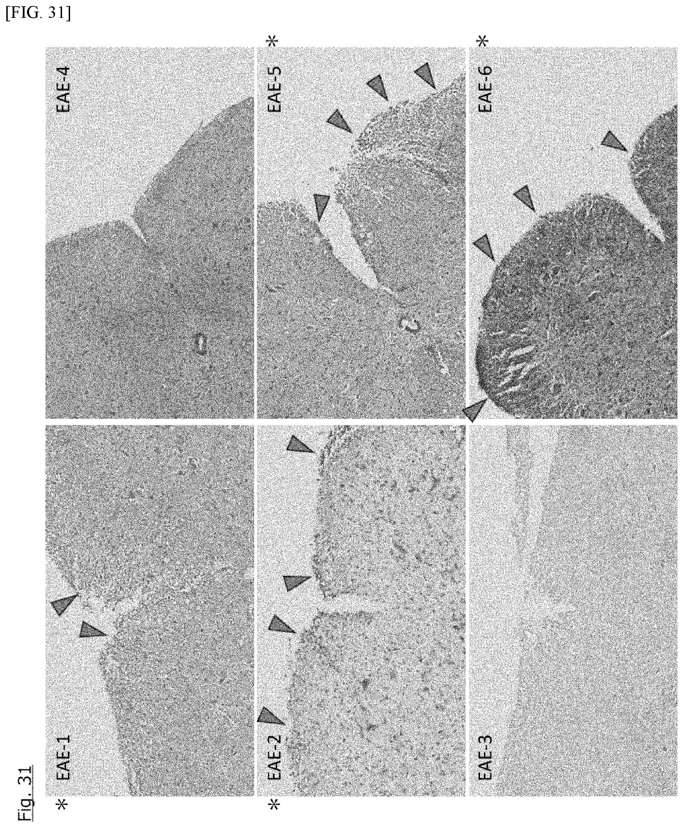

FIG. 30 shows %ID/g of individuals in the EAEs model group and the negative control group (CTL group). FIG. 31 shows images of HE-stained spinal cords of individuals of the EAE model group. Individuals marked with an asterisk in FIG 30 and 31 are those in which accumulation of the [¹¹C]HCA2969 tracer was observed in the spinal cord. In EAE1, EAE2, EAE5, and EAE6 individuals exhibiting accumulation of the [¹¹C]HCA2969 tracer in the spinal cord, lymphocyte infiltration was observed in the spinal cord tissue by HE staining.

These results indicate that a [¹¹C]HCA2969 tracer can evaluate inflammation accompanied by lymphocyte infiltration not only in myocardial tissue but also in other tissues.

## Claims

1. A radioactive positron-emitting nuclide carbon-11 labeled compound ([¹¹C] labeled compound) represented by the following general formula (I), or a pharmaceutically acceptable salt thereof: 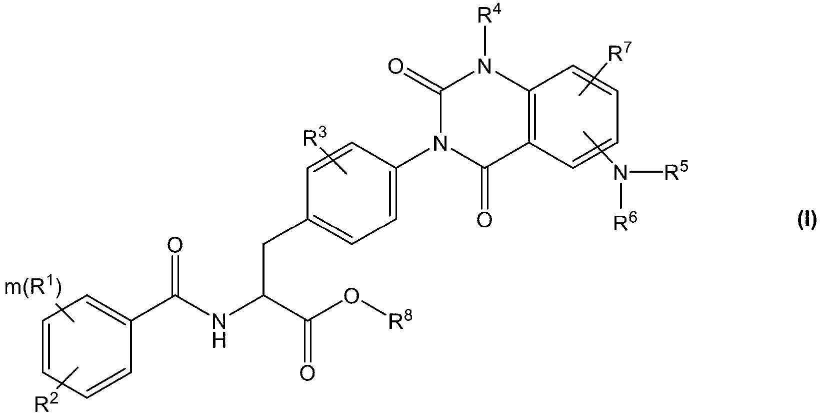 (wherein,
R¹ is a chlorine atom.
m is an integer from 1 to 3.
At least one of R², R³, R⁴, R⁵, R⁶, and R⁷ is a linear or branched alkyl group having 1 to 6 carbon atoms, wherein the alkyl group has a radioactive positron-emitting nuclide carbon-11 [¹¹C], and the rest are hydrogen atoms.
R⁸ is a hydrogen atom or a linear or branched alkyl group having 1 to 6 carbon atoms.)

2. The [¹¹C] labeled compound or the pharmaceutically acceptable salt thereof according to claim 1, wherein the [¹¹C] labeled compound is represented by the following general formula (II): 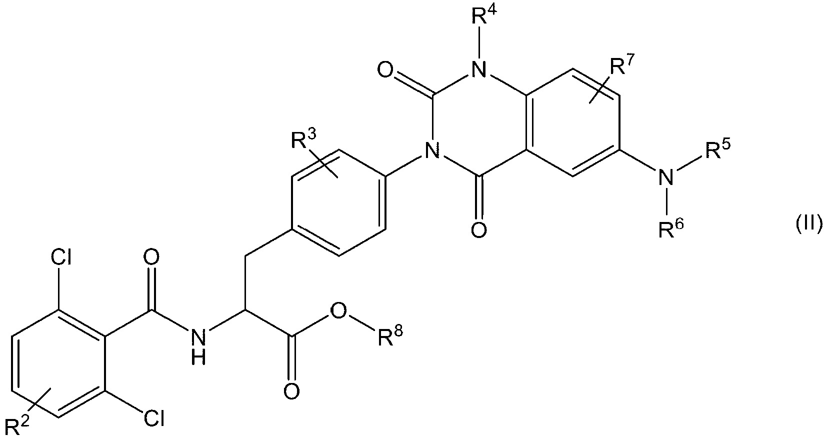 (wherein,
R², R³, R⁴, R⁵, R⁶, R⁷ and R⁸ are the same as described above.)

3. The [¹¹C] labeled compound or the pharmaceutically acceptable salt thereof according to claim 1, wherein the [¹¹C] labeled compound is represented by the following formula (1) or (2):

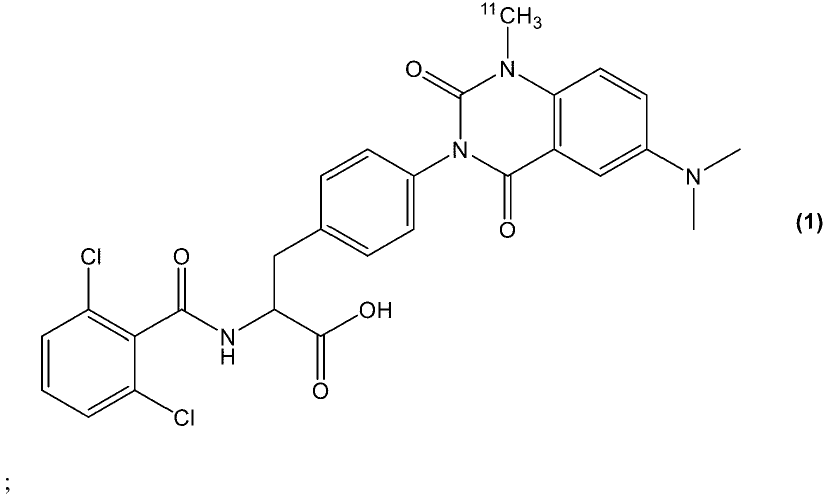

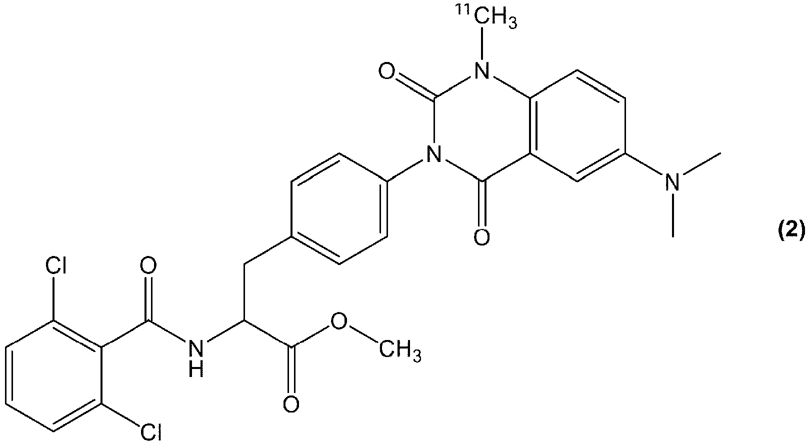

4. A radioactive composition containing the [¹¹C] labeled compound or the pharmaceutically acceptable salt thereof according to claim 1.

5. The radioactive composition according to claim 4, used for detecting a lymphocytic inflammatory disease.

6. The radioactive composition according to claim 5, wherein the lymphocytic inflammatory disease is myocarditis or myelitis (provided that autoimmune myocarditis with myosin as an antigen is excluded from the myocarditis).

7. The radioactive composition according to claim 5, wherein the myocarditis is myocarditis that develops in a manner dependent on binding of Vcam-1 and VLA-4.

8. The radioactive composition according to claim 5, wherein the myocarditis is myocarditis caused by an immune checkpoint inhibitor.

9. The radioactive composition according to claim 4, used for determining a therapeutic effect against myocarditis.

10. The radioactive composition according to claim 4, used for predicting a therapeutic effect against myocarditis.

11. The radioactive composition according to claim 4, used in patients with myocarditis or patients suspected of having myocarditis to predict a therapeutic effect of treatment with a VLA-4 inhibitor and to determine whether to administer said treatment to patients in whom the treatment is determined to be effective.
